(19) Europäisches Patentamt
European Patent Office
Office européen des brevets

(11) Publication number: **0 050 019**
**B1**

# (12) EUROPEAN PATENT SPECIFICATION

(45) Date of publication of patent specification: **09.04.86**

(21) Application number: **81304703.2**

(22) Date of filing: **09.10.81**

(51) Int. Cl.[4]: **C 07 C 69/736,**
C 07 C 69/738, C 07 C 69/712,
C 07 C 79/35, C 07 C 59/68,
C 07 C 43/295,
C 07 C 103/178, C 07 C 93/14,
C 07 C 49/255, A 01 N 39/02
// C07C69/734, C07D319/06

## (54) 3-Keto-4-(4'-aromatically substituted-phenoxy) compounds, their 3- alkylated enol and 2,3-hydrogenated derivatives and the use thereof for the control of weeds.

(30) Priority: **14.10.80 US 196795**
**05.06.81 US 270938**
**04.09.81 US 299413**

(43) Date of publication of application:
**21.04.82 Bulletin 82/16**

(45) Publication of the grant of the patent:
**09.04.86 Bulletin 86/15**

(84) Designated Contracting States:
**AT BE CH DE FR GB IT LI NL**

(56) References cited:
**DE-A-2 715 284**
**FR-A-1 559 649**
**GB-A-1 098 112**
**GB-A-1 098 113**

(73) Proprietor: **ZOECON CORPORATION**
**975 California Avenue**
**Palo Alto California 94304 (US)**

(72) Inventor: **Lee, Shy-Fuh**
**830 Lois Avenue**
**Sunnyvale California 94087 (US)**

(74) Representative: **Harrison, David Christopher et al**
**MEWBURN ELLIS & CO 2/3 Cursitor Street**
**London EC4A 1BQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The novel compounds of the present invention are represented by the following formulas A', B', C', and D':

(A') 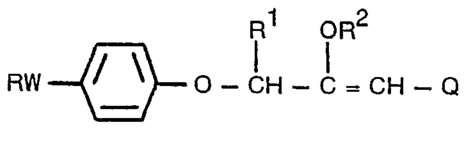 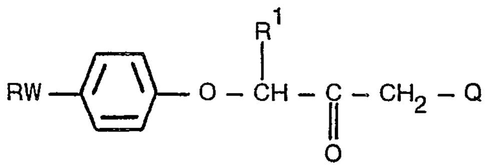 (B')

(C') 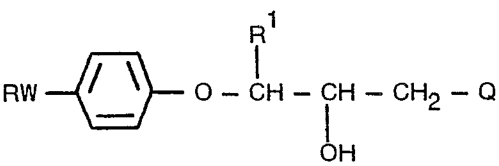 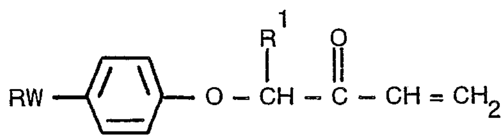 (D')

wherein

R is

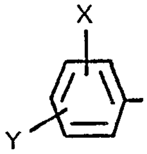, 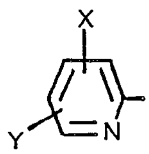, 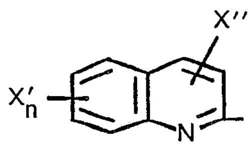 or 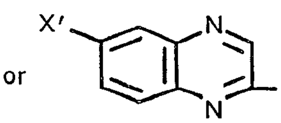,

W is O, S, NH or $CH_2$;

each of X and Y is independently selected from hydrogen, bromo, chloro, fluoro, lower alkyl, lower alkoxy, lower alkoxycarbonyl, nitro, cyano, trifluoromethyl, chlorodifluoromethyl, fluoromethyl, chloromethyl, difluoromethoxy;

n is 1 or 2;

each of X' and X'' is independently selected from hydrogen, fluoro, chloro, bromo, iodo, trifluoromethyl, methoxy or nitro, provided that both X' and X'' cannot be trifluoromethyl, methoxy or nitro;

$R^1$ is hydrogen or lower alkyl;

$R^2$ is lower alkyl;

Q is

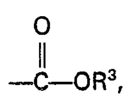, 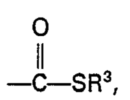, 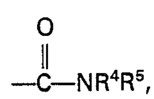,

$—CH_2—X^3$ or $—CH_2—OR^6$ in which,

$R^3$ is hydrogen, lower alkyl, lower haloalkyl, lower cyanoalkyl, lower alkenyl, lower haloalkenyl, lower alkynyl, lower alkoxyalkyl, lower dialkyaminoalkyl, lower alkylthioalkyl, lower alkylsulfinylalkyl, lower alkylsulfonylalkyl, tetrahydrofuranylmethyl, tetrahydropyranylmethyl, benzyl, halobenzyl, lower alkylbenzyl, lower alkoxybenzyl, phenyl, halophenyl, lower alkoxyphenyl, lower alkylphenyl, cycloalkyl, cycloalkylalkyl, N-alkylideneamino, sodium, potassium, magnesium or calcium;

each of $R^4$ and $R^5$ is independently selected from hydrogen, lower alkyl, lower hydroxyalkyl, lower alkoxyalkyl or lower alkylsulfonyl;

$X^3$ is bromo chloro fluoro or iodo; and

$R^6$ is hydrogen or acyl.

In the description and Claims hereinafter, each of $R—R^6$, n, Q, W, X, X', X'', $X^3$ and Y is as defined above unless otherwise specified.

In DE—A—2715284 various herbicidal compounds are shown which have some structure in common with certain of the present compounds, particularly in that a (substituted pyridyloxy) phenoxy substituent is present on a 5-member fatty acid chain. In GB—A—1098112 compounds having formulae equally related to certain of the present ones are disclosed to be useful in the treatment of atherosclerosis.

The compounds of formula (A') wherein Q is

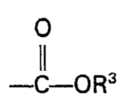

can be produced by the reaction of a phenol of formula (I).

(I) 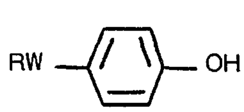

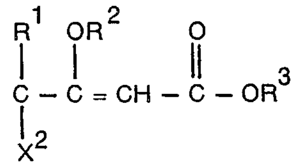 (II)

with a 4-halo compound of formula (II) in the presence of a base such as alkali metal hydroxide or alkali metal carbonate. The reaction is generally conducted at about room temperature to reflux temperature in an organic solvent such as dimethylformamide (DMF), acetone, tetrahydrofuran (THF), dimethylacetamide (DMA), dimethylsulfoxide (DMSO), or the like using about equimolar amounts of base, a phenol of formula (I) and a 4-halo compound of formula (II). $X^2$ represents bromo, chloro or iodo.

The thio compounds of formula (A′) can be prepared starting with an acid of formula (A′) which is converted into the acid halide and then reacted with a mercaptan. Alternatively, a phenol of formula (I) is reacted with a 4-halo thiolester of the general formula (II) above in the presence of base.

An amide of formula (A′) can be prepared by the reaction of an acid halide of an acid of formula (A′) with the appropriate amine.

An alcohol of formula (A′) wherein Q is $—CH_2—OR^6$ can be prepared by reduction of an acid or ester of formula (A′) using, for example, lithium borohydride or lithium aluminumhydride in ether or THF at a low temperature. Esters of an alcohol of formula (A′) can be prepared by the reaction of an acyl halide or acyl anhydride in pyridine at about room temperature or lower.

A halide of the present invention of formula (A′) wherein Q is $—CH_2—X^3$ can be prepared by reaction of an alcohol of formula (A′) wherein Q is $—CH_2—OH$ with phosphorus tribromide, phosphorus trichloride, or triphenylphosphine dihalide in acetonitrile.

In another embodiment of the present invention, there is provided 3-oxo derivatives of formula (B′) which can be derived from the novel compounds of formula (A′).

The compounds of formula (B′) are useful for the control of weeds.

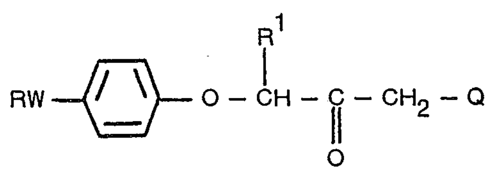 (B′)

A compound of formula (B′) can be prepared by the reaction of a 3-alkoxy compound of formula (A′) such as a 3-methoxy compounds of formula (A′) with an acid such as dilute perchloric acid or acetic acid in an organic solvent such as methylenedichloride, carbon tetrachloride or THF. The reaction is usually conducted at about room temperature or lower.

In another embodiment of the present invention, there is provided compounds of formula (C′) which can be prepared by selective reduction of a compound of formula (B′) using sodium borohydride in alcohol at low temperature.

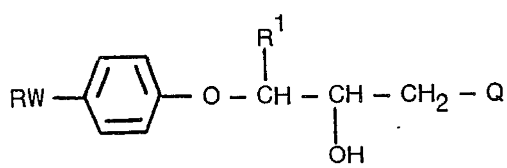 (C′)

The alcohols of formula (C′) and carboxylic esters thereof are useful for the control of weeds.

In another embodiment of the present invention, there is provided compounds of formula (D′) which are useful for the control of weeds.

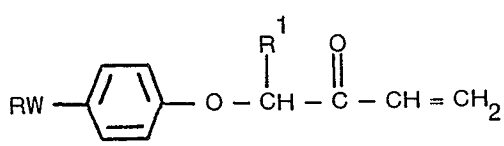 (D′)

The compounds of formula (D′) can be prepared by treating an alcohol of formula (A′) wherein Q is $—CH_2—OH$ with perchloric acid.

Carboxylic esters of formula (B′) wherein Q is

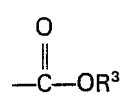

can be prepared by the following outlined method also:

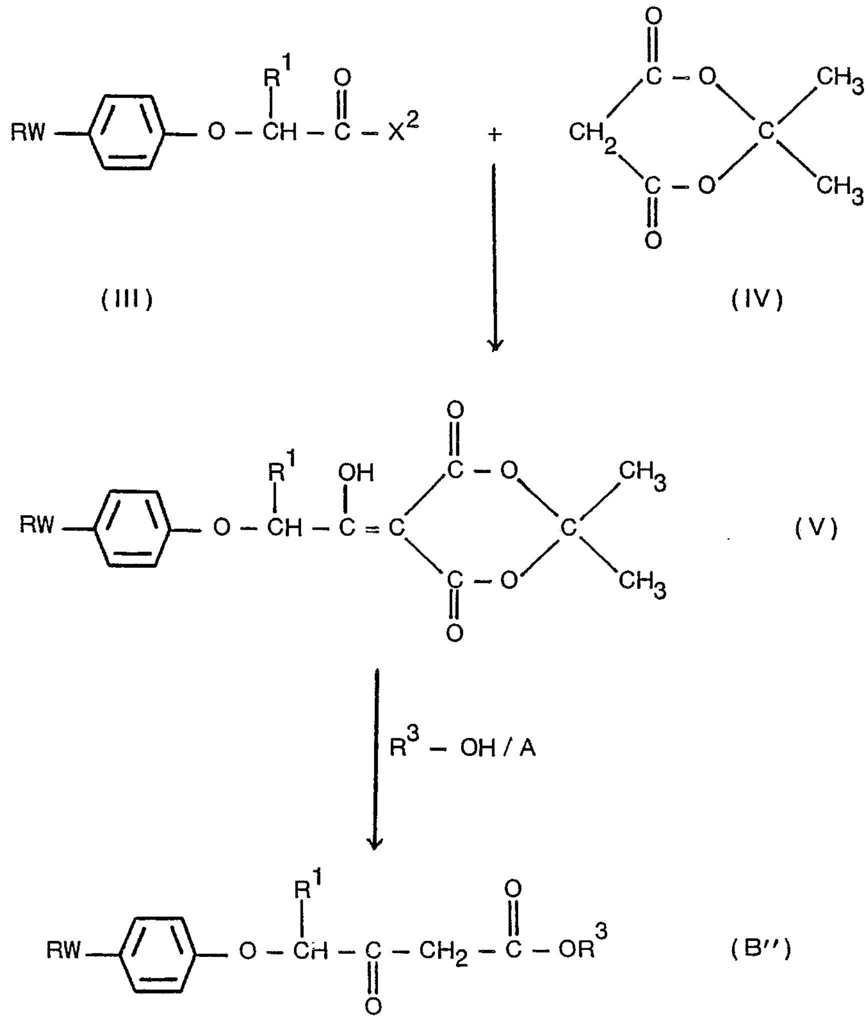


(III) (IV) (V) (B'')

The following terms have the meaning indicated as used herein and the appended claims.

The term "lower alkyl" refers to an alkyl group of one to six carbon atoms.

The term "lower alkoxy" refers to an alkoxy group of one to six carbon atoms.

The term "lower alkoxycarbonyl" refers to an alkoxycarbonyl group of two to six carbon atoms.

The term "acyl" refers to a carboxylic acyl group such as lower aliphatic acyl group of one to six carbon atoms or aryl acyl group of six to twelve carbon atoms. Typical acyl groups include acetyl, benzoyl, p-chlorobenzoyl, and the like.

The term "lower alkenyl" refers to an alkenyl group of two to six carbon atoms with monounsaturation such as allyl and n-but-2-enyl.

The term "lower alkynyl" refers to an alkynyl group of two to six carbon atoms with monounsaturation such as propargyl.

The term "cycloalkyl" refers to a cycloalkyl group of three to six carbon atoms.

The term "cycloalkyl" refers to a cycloalkyl group of four to seven carbon atoms.

The term "halo" refers to a bromo, chloro or fluoro group.

The compounds of formula A', B', C', D' are useful for the control of weeds using pre- and/or postemergent treatments. The compounds can be applied in the form of dusts, granules, solutions, emulsions, wettable powders and suspentions. Application of a compound of the present invention is made according to conventional procedure using from about one-half or less to ten pounds per acre (0.55 or less to 11 kg/ha). The application of a compound of the present invention to the "locus" of the weeds includes application to the seeds, the plant (weed) or parts of the plant, or the soil. Methods of preparing herbicidal formulations which can be used with a compound of the present invention are described in the literature along with suitable liquid and solid carrier materials such as in U.S. Patent 4,192,669 and 4,163,661 which are incorporated herein by reference. While some of the compounds of the present invention have activity on broad leaf plants, the compounds, in general, demonstrate a higher level of herbicidal activity on the grass weeds.

The optimum usage of a compound of the present invention is readily determinable by one of ordinary skill in the art using routine testing such as greenhouse testing and small plot testing.

The term "herbicide", as used herein, refers to an active ingredient which modifies the growth of plants because of phytotoxic or plant growth regulating properties so as to retard the growth of the plant or damage the plant sufficiently to kill it.

The compounds of the present invention demonstrate selective activity as herbicides against grass weeds. Crops such as cotton, sugar beet, soybeans and squash show excellent tolerance. The compounds of the present invention, in general, show higher level of herbicidal activity when the postemergent method of application is used. Grass plant (weed) species on which the compounds of the present invention show effective herbicidal activity include shattercane, crabgrass, sprangletop, wild oats, bermudagrass, tall fescue, rice, wheat, barley, corn, blue panicum, foxtails, rough bluegrass, winter rye, annual ryegrass, watergrass and Johnsongrass. It appears to be most effective to apply the active compound prior to the heading stage of the grass weed.

The compounds of the present invention, in view of their broadspectrum grass weed herbicidal activity, can be advantageously combined with broadleaf weed herbicides for broadspectrum postermergence weed control in most broadleaf crops. Examples of herbicides which can be combined with a compound of the present invention include glyphosate, bentazone, diuron, paraquat, 2,4—D, 2,4—DB, diquat, endothal, dinoseb, dicamba, norflurazon, nitrofen, cyanozine, methazole, mefluidide, metribuzin, cycloate, fluometuron, linuron, dalapon, bifenox and alachlor for controlling a broad spectrum of weeds.

The compounds of the present invention are useful for application to sugarcane in order to increase the sucrose content of the sugarcane.

The following examples are provided to illustrate the practice of the present invention. Temperature is given in degrees centigrade. All parts are by weight unless otherwise indicated. RT means room temperature.

### Example 1

A mixture of 4-(2-chloro-4-trifluoromethylphenoxy) phenol (1.16 g), ethyl 4-bromo-3-methoxy-2-pentenoate (1.74 g) and potassium carbonate (0.73 g) in DMF (5 ml.) is heated at about 130° for 2 hours. Thereafter, DMF is removed. The residue is filtered and washed with methylene dichloride. The filtrate is washed, dried and evaporated to dryness. The oily residue is subjected to prep. thin layer chromatography using 20% ethylacetate/hexane to yield the ethyl ester of 4-[4-(2-chloro-4-trifluoromethylphenoxy) phenoxy]-3-methoxy-2-pentenoic acid, MS m/e 444 ($M^+$).

### Example 2

A mixture of 4-(2-chloro-4-trifluoromethylphenoxy) phenol (6.9 mmol.), methyl 4-bromo-3-methoxy-2-pentenoate (8.9 mmol.) and potassium carbonate (1.5 equis.) in acetone (15 ml.) is refluxed for 48 hours. After filtration, the filtrate was concentrated and the residue chromatographed on silica gel using 20% ethylacetate/hexane to give the methyl ester of 4-[4-(2-chloro-4-trifluoromethylphenoxy)phenoxy]-3-methoxy-2-pentenoic acid.

Some compounds of formula A′ which are prepared according to the procedure of the foregoing examples are as follows (Table I and II).

TABLE I

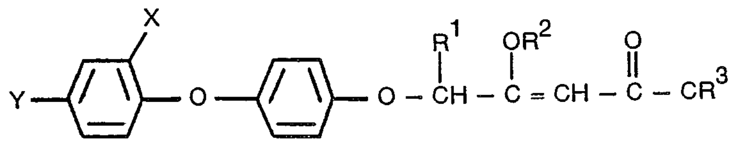

| X | Y | $R^1$ | $R^2$ | $R^3$ |
|---|---|---|---|---|
| H | $CF_3$ | Me | Me | Me |
| H | $CF_3$ | Et | Me | Me |
| Cl | $CF_3$ | Et | Me | Me |
| Cl | $CF_3$ | Me | Et | Me |
| H | $OCF_3$ | Me | Me | Me |
| Cl | $OCF_3$ | Me | Me | Me |
| Cl | Cl | Me | Me | Et |
| $NO_2$ | $CF_3$ | Me | Me | Me |
| H | Br | Me | Me | Et |
| Me | OMe | Me | Me | Me |

TABLE II

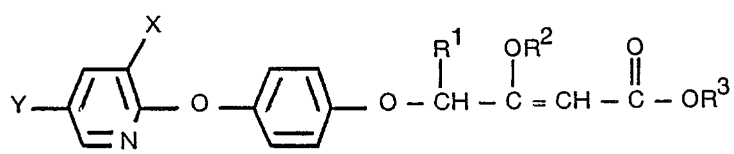

| X | Y | $R^1$ | $R^2$ | $R^3$ |
|---|---|---|---|---|
| Cl | Cl | Me | Me | Me |
| H | $CF_3$ | Me | Me | Me |
| Cl | $CF_3$ | Me | Me | Me |
| H | $CHF_2$ | Me | Me | Me |
| H | $CClF_2$ | Me | Me | Me |
| Me | $CF_3$ | Et | Me | Et |
| F | $CF_3$ | Me | Me | Me |
| H | Br | Me | Me | Me |
| Cl | Cl | Me | Et | Et |
| Cl | Cl | Et | Et | Et |

Example 3

A mixture of the methyl ester of 4-[4-(2-chloro-4-trifluoromethylphenoxy) phenoxy]-3-methoxy-2-pentenoic acid (0.5 g), 35% aqueous perchloric acid (5 ml.) and methylene dichloride (5 ml.) is stirred at RT

for 4 days. Then the solution is extracted with methylene dichloride. The combined extracts are washed, dried and evaporated. The residue is purified by prep. thin layer chromatography using 20% ethyl acetate/hexane to yield methyl 4-[4-(2-chloro-4-trifluoromethylphenoxy) phenoxy]-3-oxopentanoate.

The product of Example 1 is treated with aqueous perchloric acid as above to yield ethyl 4-[4-(2-chloro-4-trifluoromethylphenoxy) phenoxy]-3-oxopentanoate, ("Compound 2") MS m/e 430 ($M^+$).

By use of the foregoing procedure, each of the compounds of Tables I and II are converted into the corresponding 3-oxo- compound of formula (F) such as methyl 4-[4-(4-trifluoromethylphenoxy)phenoxy]-3-oxopentanoate, methyl 4-[4-(4-trifluoromethoxy-phenoxy)phenoxy]-3-oxopentanoate, ethyl 4-[4-(2,4-dichlorophenoxy)phenoxy]-3-oxopentanoate, MS m/e 396 ($M^+$), methyl 4-[4-(4-dichlorophenoxy)phenoxy]-3-oxopentanoate, methyl 4-[4-(3,5-dichloropyridyloxy)phenoxy]-3-oxopentanoate and methyl 4-[4-(5-trifluoromethylpyridyloxy)phenoxy]-3-oxopentanoate.

Example 4

A mixture of 4-(2-nitro-4-trifluoromethylphenoxy) phenol (9.36 mm)., ethyl 4-bromo-3-methoxy-2-pentenoate (12 mm.) and $K_2CO_3$ (14 mm.) in acetone (20 ml.) is refluxed for 24 hours. After filtration, the filtrate is concentrated and the oily residue chromatographed on silica gel to yield ethyl 4-[4-(2-nitro-4-trifluoromethylphenoxy)phenoxy]-3-methoxy-2-pentenoate.

A solution of the above ethyl ester in 35% perchloric acid and water is stirred at RT for 3 days and then worked up to give ethyl 4-[4-(2-nitro-4-trifluoromethylphenoxy) phenoxy-3-oxopentanoate.

Example 5

To a mixture of lithium aluminum hydride (200 mg) and anhydrous ether (5 ml.) is added dropwise at 0°, a mixture of ethyl 4[4-(2-chloro-4-trifluoromethylphenoxy)phenoxy]-3-methoxy-2-pentenoate (400 mg) and ether (5 ml.). After addition is complete, the mixture is stirred. Wet ether and water is added to destroy excess hydride. Filtration and evaporation of filtrate gives 4-[4-(2-chloro-4-trifluoromethylphenoxy)phenoxy]-3-methoxy-2-penten-1-ol.

Example 6

A mixture of ethyl 4-[4-(2-chloro-4-trifluoromethylphenoxy)phenoxy]-3-methoxy-2-pentenoate (10 mmol), ethanol (20 ml.) and 1N Na OH (20 ml.) is prepared and then heated at reflux for a few hours to complete the reaction. Removal of solvent gives the sodium salt of 4-[4-(2-chloro-4-trifluoromethylphenoxy)phenoxy]-3-methoxy-2-pentenoic acid. The free acid is obtained by acidification of the salt using dilute $H_2SO_4$, and extraction with methylene dichloride and evaporation. Other acid salts can be prepared by titrating the free acid with an organic solution of the base, e.g., methanolic solution of potassium methoxide and the like.

Example 7

Thionyl chloride (20 ml.) is added to 4-[4-(2-chloro-4-trifluoromethylphenoxy)phenoxy]-3-methoxy-2-pentenoic acid (10 mm.) in anhydrous ether (10 ml.). The mixture is refluxed for a few hours. Then, ether and excess thionyl chloride is removed by vacuum to yield 4-[4-(2-chloro-4-trifluoromethylphenoxy)phenoxy]-3-methoxy-2-pentenoyl chloride.

Example 8

To a mixture of 4-[4-(2-chloro-4-trifluoromethylphenoxy) phenoxy]-3-methoxy-2-pentenoyl chloride (10 mm.) and ether (10 ml.) is added methyl mercaptan (12 mm.) and pyridine (1 ml.) at about −20°. The mixture is stirred for about 30 minutes and then allowed to rise to about 0° and stand for about 2 hours. The mixture is then diluted with ether and water and the ether phase separated, washed, dried and solvent removed to give the methylthiol ester of 4-[4-(2-chloro-4-trifluoromethylphenoxy) phenoxy]-3-methoxy-2-pentenoic acid.

Example 9

A mixture of 4-[4-(2-chloro-4-trifluoromethylphenoxy) phenoxy]-3-methoxy-2-pentenoyl chloride (15 mm) and THF (15 ml.), at 0°, is prepared and then slight excess of methylamine introduced. The reaction mixture is allowed to stand under nitrogen for about one hour. Then the reaction is allowed to rise to RT and solvent removed. The residue is taken up in methylene dichloride, washed, dried and solvent removed under vacuum to yield N-methyl 4-[4-(2-chloro-4-trifluoromethylphenoxy) phenoxy]-3-methoxy-2-pentenamide.

Example 10

A mixture of ethyl 3-oxopentanoate (20 g) and trimethylformate (5 eq., 94 ml.) in the presence of 6 g of Amberlyst-15 (trademark of Rohm & Haas Corp.) is stirred at RT overnight. After filtration, the filtrate is concentrated and then distilled to give ethyl 3-methoxy-2-pentenoate (19.5 g).

A mixture of the above ethyl ester, N-bromosuccinimide (26.3 g), benzoyl peroxide (0.3 g.) and carbon tetrachloride (100 ml.) is heated to reflux and irradiated with 150 watt flood lamp until substantially clear solution is obtained. Thereafter, the reaction is filtered and the filtrate then concentrated and distilled (78°/2 mm) to give ethyl 4-bromo-3-methoxy-2-pentenoate.

Example 11

(A) Following the procedure of Example 1, 4-(2-nitro-4-trifluoromethylphenylamino) phenol is reacted with ethyl 4-bromo-3-methoxy-2-pentenoate to give ethyl 4-[4-(2-nitro-4-trifluoromethylphenylamino)phenoxy]-3-methoxy-2-pentenoate.

(B) Following the procedure of Example 3, the product of part (A) is treated with aqueous perchloric acid to give ethyl 4-[4-(2-nitro-4-trifluoromethylphenylamino)phenoxy]-3 oxopentanoate, MS m/e 512 ($M^+$).

In the same way, there is prepared methyl 4-[4-(2-nitro-4-chlorophenylamino)phenoxy]-3-methoxy-2-pentenoate and methyl 4-[4-(2-nitro-4-chlorophenylamino)phenoxy]-3-oxopentanoate starting with 4-(4-chloro-2-nitrophenylamino) phenol and methyl 4-bromo-3-methoxy-2-pentenoate.

Example 12

A spray solution of water/acetone (1:1) and surfactant (1%) was prepared containing (1) the compound of Example 1 (Compound 1) and (2) the second compound of Example 3 (Compound 2) and sprayed (equivalent to 10 lb/acre) on seedlings of foxtail (*Setaria viridis*) and watergrass (*Echinochloa crusgalli*). Observation two weeks after spraying Compound 1 and 2 showed 100% herbicidal activity for each of test Compounds 1 and 2. Testing of Compounds 1 and 2 showed no injury to soybeans at 10 lb/acre.

Pre-emergent herbicidal activity of Compound 2 was tested on foxtail, watergrass, shattercane, and wild oats. The average activity was 86% for the four species.

Example 13

To a slurry of $LiAlH_4$ (100 mg) in anhydrous ether (20 ml) is added dropwise at 0°, a solution of ethyl 4-[4-(4-trifluoromethylphenoxy)phenoxy]-3-methoxy-2-pentenoate (500 mg) in ether. After addition is complete, the reaction mixture is stirred for about 20 minutes. Excess of the hydride is decomposed using wet ether and water. Filtration and evaporation of filtrate gives the C—1 alcohol, 4-[4-(4-trifluoromethylphenoxy)phenoxy]-3-methoxy-2-penten-1-ol, MS m/e 440 ($M^+$).

Example 14

To a slurry of $LiAlH_4$ (200 mg) in anhydrous ether (15 ml) is added at 0° a solution of ethyl 4-[4-(4-trifluoromethylphenoxy)phenoxy]-3-oxopentanoate (400 mg) in ether (5 ml). After addition is complete, the reaction mixture is stirred at RT for about one hour. Excess of the hydride is decomposed using wet ether and water, the mixture is filtered and filtrate concentrated. The oily product is purified by prep. thin layer chromatography to give 4-[4-(4-trifluoromethylphenoxy)phenoxy]-pentane-1,3-diol, MS m/e 500 ($M^+$).

Example 15

To a solution of ethyl 4-[4-(2-nitro-4-chlorophenoxy) phenoxy]-3-oxopentanoate (600 mg) in absolute ethanol (10 ml) is added at 0°, $NaBH_4$ (300 mg). After addition is complete, the reaction mixture is stirred for about 20 minutes. Then the solution is taken up in $CH_2Cl_2$, washed with brine, dried and evaporated to give ethyl 4-[4-(2-nitro-4-chlorophenoxy) phenoxy]-3-hydroxypentanoate.

Example 16

To a slurry of $LiAlH_4$ (400 mg) in anhydrous ether (20 ml) is added dropwise at 0°, a solution of ethyl 4-[4-(4-trifluoromethylphenoxy)phenoxy]-3-methoxy-2-pentenoate (1.2 g) in ether (10 ml). After addition is complete, the reaction mixture is stirred for about 10 minutes. Excess hydride is decomposed with wet ether and water. After filtration, the filtrate is concentrated to give the alcohol, 4-[4-(4-trifluoromethylphenoxy) phenoxy]-3-methoxy-2-penten-1-ol.

The alcohol (600 mg) is stirred with 5 ml of 35% $HClO_4$ in $CH_2Cl_2$ (5 ml) for about 20 minutes. The solution is then extracted with $CH_2Cl_2$ followed by washing, drying and evaporation. The product is then purified by prep. thin layer chromatography to give 4-[4-(4-trifluoromethylphenoxy)phenoxy]-3-oxo-1-pentene, MS m/e 336 ($M^+$).

Example 17

To a solution of ethyl 4-[4-(4-trifluoromethylphenoxy) phenoxy]-3-oxopentanoate (450 g) in ethanol (5 ml) is added $NaBH_4$ (150 mg) at 0°. The resulting mixture is then stirred at 0° for about 10 minutes. The mixture is diluted with $CH_2$ $Cl_2$, washed with saturated NaCl solution, dried and evaporated to dryness to give ethyl 4-[4-(4-trifluoromethylphenoxy)phenoxy]-3-hydroxypentanoate, in quantitative yield, MS m/e 398 ($M^+$).

Example 18

The process of Example 17 is repeated using ethyl 4-[4-(2-chloro-4-trifluoromethylphenoxy)phenoxy]-3-oxopentanoate (400 mg) and $NaBH_4$ (100 mg) to give ethyl 4-[4-(2-chloro-4-trifluoromethylphenoxy)phenoxy]-3-hydroxypentanoate, MS m/e 432 ($M^+$).

Example 19

To a slurry solution of $LiAlH_4$ (200 mg) in anhydrous ether (5 ml) is added dropwise at 0°, a solution of ethyl 4-[4-(2-chloro-4-trifluoromethylphenoxy)phenoxy]-3-methoxy-2-pentenoate (400 mg) in anhydrous

ether (5 ml). After addition is complete, the reaction mixture is stirred at 0° for about 10 minutes. Excess hydride is decomposed with wet ether and water. The mixture is filtered and filtrate washed, dried and evaporated to give 4-[4-(2-chloro-4-trifluoromethylphenoxy) phenoxy]-3-methoxy-2-penten-1-ol.

The foregoing alcohol (380 mg) is reacted with 35% $HClO_4$ (4 ml) in $CH_2Cl_2$ (4 ml) at RT for 24 hours. The mixture is then extracted with $CH_2Cl_2$ and the combined extracts washed, dried and concentrated to dryness. The product is purified by prep. thin layer chromatography to give 4-[4-(2-chloro-4-trifluoromethylphenoxy)phenoxy]-3-oxo-1-pentene, MS m/e 396 ($M^+$).

Example 20

A solution of 2-[4-(4-trifluoromethylphenoxy)phenoxy]-propionyl chloride (1.5 g) and methylene chloride (5 ml) is added dropwise at 0° to a solution of 2,2-dimethyl-1,3-dioxane-4,6-dione (680 mg) in methylene chloride (20 ml) containing pyridine (0.7 ml). The resulting mixture is stirred at RT for 2 hours. The reaction mixture is then poured into water, acidified with dilute HCl and extracted with methylene chloride. The combined extracts are washed with brine, dried and evaporated to give 2-[4-(4-trifluoromethylphenoxy)phenoxy]-propionyl meldrum acid, an oil.

The above meldrum acid is treated with n-butanol (20 ml) at 120° for about 4 hours. After aqueous workup, the oily concentrate is purified by prep. thin layer chromatography on silica gel to give n-butyl 4-[4-(4-trifluoromethylphenoxy) phenoxy]-3-oxopentanoate, MS m/e 424 ($M^+$).

Example 21

A mixture of 2-nitro-3-trifluoromethyl-4'-hydroxydiphenylamine (1 g), ethyl 4-bromo-3-methoxy-2-pentenoate (990 mg) and potassium carbonate (694 mg) in acetone (10 ml) is refluxed for 24 hours. After filtration and concentration, the crude product is purified by prep. thin layer chromatography on silica gel to give ethyl 4-[4-(2-nitro-4-trifluoromethylanilino)phenoxy]-3-methoxy-2-pentenoate which is treated with 35% $HClO_4$ in methylene chloride to give ethyl 4-[4-(2-nitro 4-trifluoromethylanilino)phenoxy]-3-oxopentanoate, MS m/e 440 ($M^+$).

Example 22

Post-emergence activity on the grasses (GR) greenfoxtail, watergrass, shattercane and wild oats was tested for the compound of Examples 13, 14, 17, 18, 20 and 21 (compounds No. 3, 4, 5, 6, 7 and 8, respectively) and on the broadleafs (BL) annual morningglory, sesboria, soybean and velvet leaf by spraying at a rate equivalent to 10 lb/acre. The average score is given in percent control.

| Compound No. | GR | BL |
|---|---|---|
| 3 | 100 | 14 |
| 4 | 96 | 8 |
| 5 | 100 | 11 |
| 6 | 100 | 3 |
| 7 | 100 | 7 |
| 8 | 100 | 0 |

Example 23

An emulsifiable concentrate was prepared having the following composition (components in percent by weight).

| | | |
|---|---|---|
| (VI) | Compound B | 27.78 |
| | Igepol CO—530 | 2.67 |
| | Tween 21 | 2.67 |
| | Tween 81 | 2.67 |
| | Corn oil | 64.21 |

Compound B is ethyl 4-[4-(4-trifluoromethylphenoxy)phenoxy]-3-oxopentanoate (90% purity). Tween 21 is non-ionic surfactant, polyoxyethylene sorbitan monolaurate and Tween 81 is polyoxyethylene sorbitan monooleate. Tween is a trademark of ICI Americas. Igepal CO—530 is a non-ionic surfactant [nonylphenoxypoly(ethyleneoxy)ethanol] of GAF, Inc.

| | | |
|---|---|---|
| (VII) | Compound C | 29.3 |
| | Toximol S | 6.4 |
| | Atlox 8916 TF | 1.6 |
| | Tenneco 500—100 | 62.7 |

Compound C is ethyl 4-[4-(4-trifluoromethylphenoxy) phenoxy]-3-hydroxypentanoate (90% purity).

A spray solution was prepared using 30 parts of concentrate VII, 950 parts of water and 28 parts of non-phytotoxic crop oil and applied to young Johnson grass at the rate of 1,1 kg per hectare (1.0 lb. per acre). The test plots were observed after two weeks. The application gave 100% control of the Johnson grass.

| | | |
|---|---|---|
| (VIII) | Compound B | 27.8 |
| | Toximol R | 3.0 |
| | Toximol S | 9.0 |
| | Tenneco 500—100 | 60.2 |

Concentrate VIII was diluted in water and applied at the rate of 2,2 kg per hectare (2.0 lb. per acre) to test specimens, in a greenhouse, of watergrass, green foxtail, shattercane, blue panicum, tallfescue, sprangletop, annual ryegrass, downy brome, wild oats, crabgrass, milo, corn, probred wheat and barley. The average herbicidal activity of two or more evaluations of each of the foregoing grass species was 80% or greater.

Toximol R and S are surfactants of the Stepan Chemical Corporation, Illinios. Atlox 8916F in a surfactant of ICI Americas, Delaware. Tenneco 500—100 is an aromatic solvent of the Tenneco Corporation.

## Example 24

Flowable formulations were prepared having the following compositions, percent by weight.

| | | | |
|---|---|---|---|
| (XI) | (A) | Compound B | 3.00 |
| | | Toximol 360A | 3.00 |
| | | Sun 7N (oil) | 30.00 |
| | (B) | Water | 60.85 |
| | | Gelvatol 20/30 | 3.00 |
| | | Kelzan | 0.15 |

Premix (A) was dispersed in high speed blender for about one minute and then Premix (B) was poured into Premix (A) while stirring at high speed. After addition of Premix (B) stirring was continued about 5 minutes.

| | | | |
|---|---|---|---|
| (X) | (A) | Compound C | 3.00 |
| | | Toximol 360A | 3.00 |
| | | Sun 7N | 30.00 |
| | (B) | Water | 60.85 |
| | | Gelvatol 20/30 | 3.00 |
| | | Darvan No. 1 | 0.15 |

Premix (A) and (B) were combined as described for flowable IX above. Concentration of active ingredient 2.7%.

Sun 7N is a non-phytotoxic oil of the Sun Chemical Company. Gelvatol 20/30 is a polyvinyl alcohol, molecular weight about 10000 of the Monsanto Company. Kelzan is a thickener agent (xanthum gum). Darvan No. 1 is a dispersant of the RT Vanderbilt Company.

Example 25

The compound, 2-[4-(4-trifluoromethylphenoxy)-phenoxy] propionyl meldrum acid (V; W is oxygen, R is 4-trifluoromethylphenyl, and $R^1$ is methyl) is reacted with the alcohol $R^3$—OH using the procedure of Example 20 to prepare the respective ester under Table III.

TABLE III

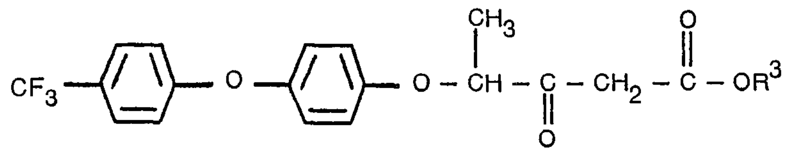

$R^3$

$-CH_2-CN$

$-CH_2-CH=CH_2$

$-CH_2-CH=C\,Cl_2$

$-CH_2-C\equiv CH$

$-CH_2-CH_2-O-CH_3$

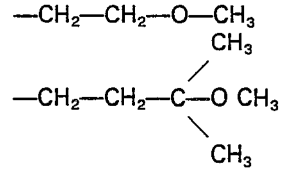

$-CH_2-CH_2-Cl$

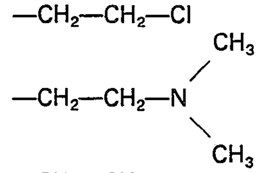

$-CH_2-CH_2-S-CH_2-CH_3$

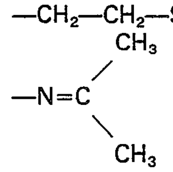

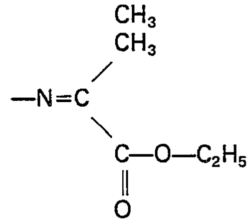

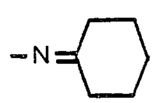

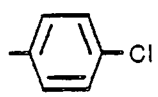

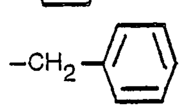

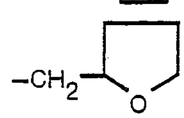

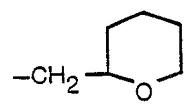

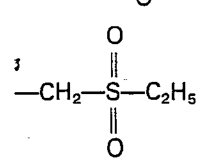

Example 26

Following the procedure of Example 17, each of the 3-oxo esters under Table III is reduced using sodium borohydride to yield the respective 3-hydroxy esters of the following formula:

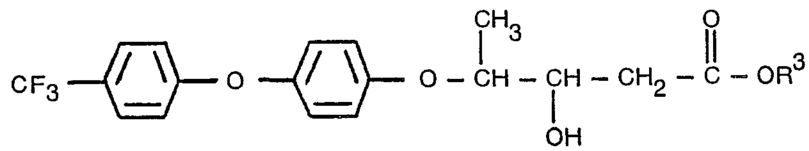

Example 27

Ethyl 4-[4-(4-trifluoromethylphenoxy)phenoxy]-3-hydrooxypentanoate is reacted with 1.1 equivalent of acetyl chloride and 1.0 equivalent of pyridine in benzene to yield ethyl 4-[4-(4-trifluoromethylphenoxy)phenoxy]-3-acetoxypentanoate. Similarly, using n-propanoyl chloride and isopropanoyl chloride yield the respective 3-n-propanoate and 3-isopropanoate.

Carbonates of the 3-hydroxy compounds of formula C' are prepared using the above procedure and 1.1 equivalents of, for example, ethyl chloroformate or methyl chloroformate. The 3-carbonates are useful herbicides for grass weeds also.

In the preparation of the 3-hydroxy compounds of formula C', in addition to reduction of the respective 3-oxo compound of formula B', the 3-hydroxy compounds such as the esters can be prepared by the following outlined method.

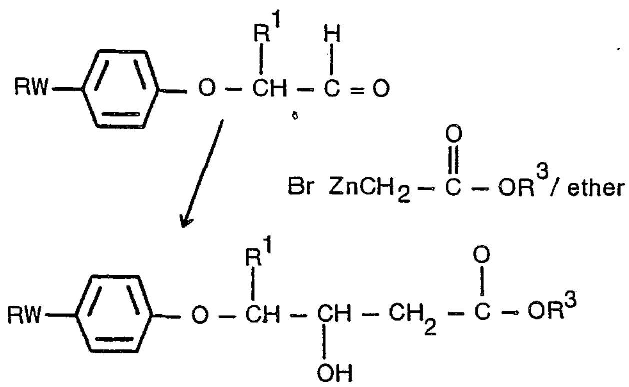


The compounds of the present invention include the isomeric forms and mixtures thereof. Thus, the invention includes the optically active isomers and racemic mixtures. Unless otherwise specified herein, the compounds described in the examples are racemic mixtures.

Example 28

Each of the compounds; methyl 4-[4-(3,5-dichloropyridyloxy)phenoxy]-3-oxopentanoate and methyl 4-[4-(5-trifluoromethylpyridyloxy)phenoxy]-3-oxopentanoate is reacted with $NaBH_4$ by the procedure of Example 17 to yield methyl 4-[4-(3,5-dichloropyridyloxy)phenoxy]-3-hydroxypentanoate and methyl 4-[4-(5-trifluoromethylpyridyloxy)phenoxy]-3-hydroxypentanoate, respectively.

Example 29

The process of Example 20 is repeated with the exception of using 2-[4-(6-chloro-2-quinolinyloxy)phenoxy]-propionyl chloride as the starting material in place of 2-[-(4-trifluoromethylphenoxy)phenoxy]-propionyl chloride to yield as the final product, n-butyl 4-[4-(6-chloro-2-quinolinyloxy)phenoxy]-3-oxopentanoate.

By using 2-[4-(6-fluoro-2-quinolinyloxy)phenoxy]-propionyl chloride as the starting material and ethyl alcohol in place of n-butyl alcohol in the process of Example 20, there is obtained ethyl 4-[4-(6-fluoro-2-quinolinyloxy) phenoxy]-3-oxopentanoate which is reduced using $NaBH_4$ in alcohol to yield ethyl 4-[4-(6-fluoro-2-quinolinyloxy) phenoxy]-3-hydroxypentanoate.

Example 30

The compound, 6-fluoro-2-(4-hydroxyphenoxy) quinoxaline, is used as the starting material in Example 1 in place of 4-(2-chloro-4-trifluoromethylphenoxy)phenol to yield ethyl 4-[4-(6-fluoro-2-quinoxalyloxy)phenoxy]-3-methoxy-2-pentenoate which is reacted with aqueous perchloric acid by the process of Example 3 to yield ethyl 4-[4-(6-fluoro-2-quinoxalyloxy)phenoxy]-3-oxopentanoate. The 3-oxo ester on reduction with $NaBH_4$ yield the 4-hydroxy ester, ethyl 4-[4-(6-fluoro-2-quinoxalyloxy)phenoxy]-3-hydroxypentanoate.

**Claims for the Contracting States: BE CH DE FR GB IT LI NL**

1. A compound selected from those of the following formulas A′, B′, C′ and D′:

(A′) 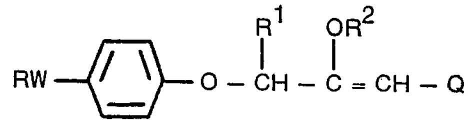

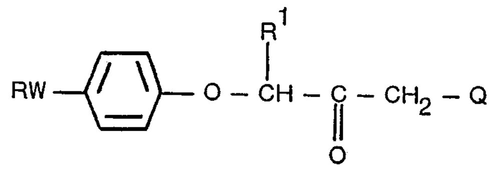 (B′)

(C′) 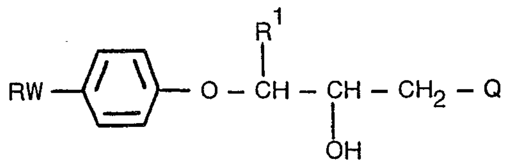

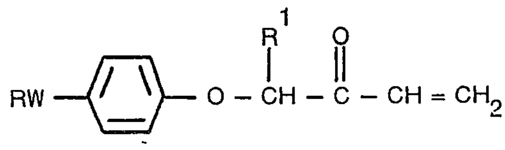 (D′)

wherein,

R is

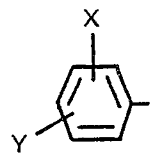 , 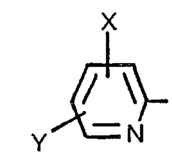 , 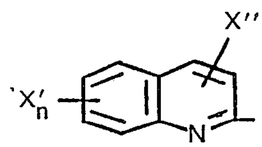 or 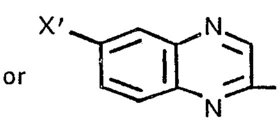 ,

W is O, S, NH or $CH_2$;

each of X and Y is independently selected from hydrogen, bromo, chloro, fluoro, lower alkyl, lower alkoxy, lower alkoxycarbonyl, nitro, cyano, trifluoromethyl, chlorodifluoromethyl, fluoromethyl, chloromethyl and difluoromethoxy;

n is 1 or 2;

each of X′ and X″ is independently selected from hydrogen, fluoro, chloro, bromo, iodo, trifluoromethyl, methoxy or nitro, provided that both X′ and X″ cannot be trifluoromethyl, methoxy or nitro;

$R^1$ is hydrogen or lower alkyl;

$R^2$ is lower alkyl;

Q is

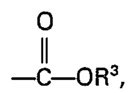, 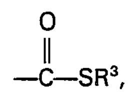, 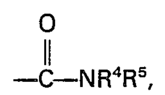,

$—CH_2—X^3$ or $—CH_2—OR^6$ in which,

$R^3$ is hydrogen, lower alkyl, lower haloalkyl, lower cyanoalkyl, lower alkenyl, lower haloalkenyl, lower alkynyl, lower alkoxyalkyl, lower dialkylaminoalkyl, lower alkylthioalkyl, lower alkylsulfinylalkyl, lower alkylsulfonylalkyl, tetrahydrofuranylmethyl, tetrahydropyranylmethyl, pyridylmethyl, benzyl, halobenzyl, lower alkylbenzyl, lower alkoxybenzyl, phenyl, halophenyl, lower alkoxyphenyl, lower alkylphenyl, cycloalkyl, cycloalkylalkyl, N-alkylideneamino, sodium, potassium, magnesium or calcium;

each of $R^4$ and $R^5$ is independently selected from hydrogen, lower alkyl, lower hydroxyalkyl, lower alkoxyalkyl or lower alkylsulfonyl;

$X^3$ is bromo, chloro, fluoro or iodo; and

$R^6$ is hydrogen or acyl.

2. A compound of the formula

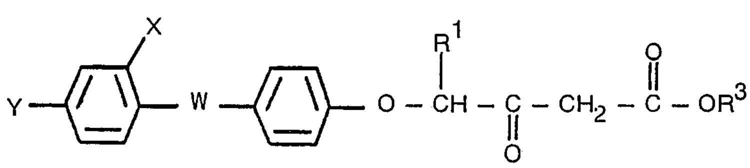

according to Claim 1.

3. A compound according to Claim 2 wherein $R^1$ is methyl and W is oxygen.

4. A compound according to Claim 3 wherein X is hydrogen or chloro and Y is trifluoromethyl or chloro.

5. A compound according to Claim 4 wherein $R^3$ is lower alkyl.

6. The methyl ester or ethyl ester of 4-[4-(4-trifluoromethylphenoxy)phenoxy]-3-oxopentanoic acid, according to Claim 5.

7. A compound according to Claim 4 wherein $R^3$ is hydrogen, sodium or potassium.

8. A compound according to Claim 4 wherein $R^3$ is cyanomethyl, propargyl, methoxyethyl, 3-methoxy-3-methylbutyl, 2-chlorethyl, N,N-dimethylaminomethyl, ethylthioethyl, ethylsulfinylethyl, ethylsulfonylethyl, benzyl, p-chlorophenyl, allyl, 3,3-dichloroprop-2-enyl, 2-tetrahydrofuranylmethyl, 2-tetrahydropyranylmethyl, N-isopropylideneamino or N-cyclohexylideneamino.

9. A compound according to Claim 2 wherein W is NH, X is nitro, Y is trifluoromethyl, $R^1$ is methyl and $R^3$ is hydrogen, sodium, potassium or lower alkyl.

10. A compound of the formula

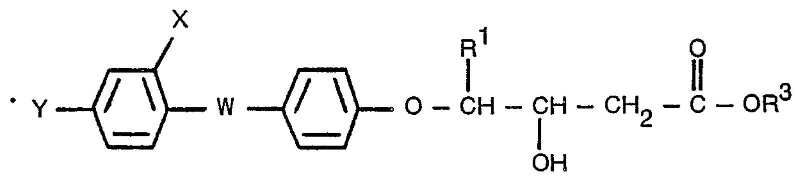

according to Claim 1.

11. A compound according to Claim 10, wherein $R^3$ is cyanomethyl, propargyl, methoxyethyl, 3-methoxy-3-methylbutyl, 2-chloroethyl, N,N-dimethylaminomethyl, ethylthioethyl, ethylsulfinylethyl, ethylsulfonylethyl, benzyl, p-chlorophenyl, allyl, 3,3-dichloroprop-2-enyl, 2-tetrahydrofuranylmethyl, 2-tetrahydropyranylmethyl, N-isopropylideneamino or N-cyclohexylideneamino, $R^1$ is methyl, W is oxygen, X is hydrogen or chloro and Y is trifluoromethyl or chloro.

12. A compound according to Claim 10, wherein W is NH, X is hydrogen or nitro, Y is trifluoromethyl, $R^1$ is methyl and $R^3$ is hydrogen, sodium, potassium or lower alkyl.

13. A compound according to Claim 10, wherein W is oxygen, X is hydrogen or chloro, Y is trifluoromethyl, $R^1$ is methyl and $R^3$ is hydrogen, sodium, potassium, or lower alkyl.

14. A compound according to Claim 13 wherein $R^3$ is methyl or ethyl.

15. A compound of the formula

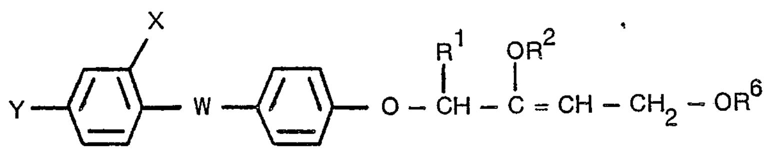

according to Claim 1, wherein each of $R^1$ and $R^2$ is methyl, W is oxygen, X is hydrogen or chloro and Y is trifluoromethyl or chloro.

16. A compound according to Claim 15 wherein each of X and $R^6$ is hydrogen.

17. A compound of the formula

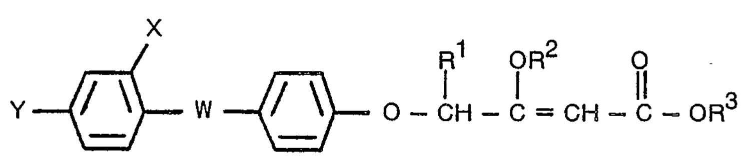

according to Claim 1, wherein each of $R^1$ and $R^2$ is methyl, $R^3$ is hydrogen, sodium, potassium or lower alkyl, X is hydrogen, chloro or nitro and Y is trifluoromethyl or chloro.

18. A compound according to Claim 17 wherein X is hydrogen or nitro and W is NH.

19. A compound according to Claim 17 wherein X is nitro.

20. A compound according to Claim 17 wherein X is hydrogen or chloro and W is oxygen.

21. A compound according to Claim 18 or Claim 20 wherein $R^3$ is methyl or ethyl.

22. A compound of the formula

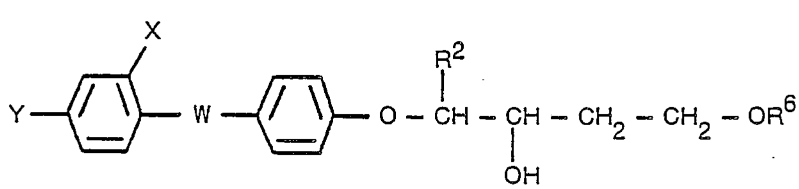

according to Claim 1, wherein $R^6$ is hydrogen, $R^2$ is methyl, W is oxygen, X is hydrogen and Y is trifluoromethyl or chloro.

23. A compound of the formula

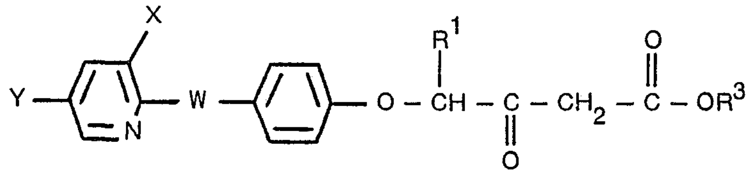

according to Claim 1.

24. A compound according to Claim 23 wherein W is oxygen, X is hydrogen, Y is trifluoromethyl, $R^1$ is methyl and $R^3$ is hydrogen, sodium, potassium, methyl or ethyl.

25. A compound according to Claim 23 wherein W is oxygen, X is chloro or bromo, Y is chloro, $R^1$ is methyl and $R^3$ is hydrogen, sodium, potassium, methyl or ethyl.

26. A compound of the formula

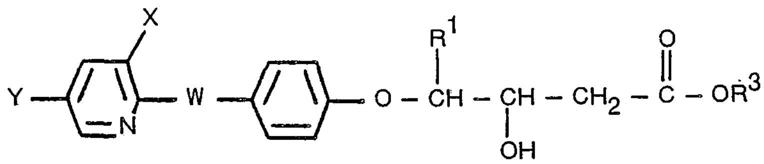

according to Claim 1.

27. A compound according to Claim 26 wherein W is oxygen, X is hydrogen, Y is trifluoromethyl, $R^1$ is methyl and $R^3$ is hydrogen, potassium, sodium or ethyl.

28. The compound of Claim 26 wherein W is oxygen, X is hydrogen, Y is trifluoromethyl, and $R^1$ and $R^3$ are both methyl.

29. A compound according to Claim 26 wherein W is oxygen, X is chloro or bromo, Y is chloro, $R^1$ is methyl and $R^3$ is hydrogen, potassium, sodium, methyl or ethyl.

30. A herbicidal composition which comprises a herbicidally effective amount of a compound of Claim 1 and a suitable carrier material.

31. A herbicidal composition which comprises a herbicidally effective amount of a compound of Claim 6 or Claim 12 and a suitable carrier material.

32. A method for the control of weeds which comprises applying to the locus of said weeds a herbicidally effective amount of a compound of Claim 1.

33. A method for the control of weeds which comprises applying to the locus of said weeds a herbicidally effective amount of a compound of Claim 6 or Claim 12.

**Claims for the Contracting State: AT**

1. A method for the control of weeds which comprises applying to the locus of the weeds a compound of the formula:

(A') 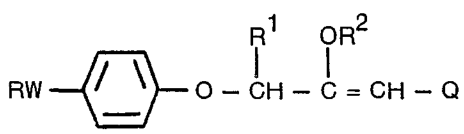

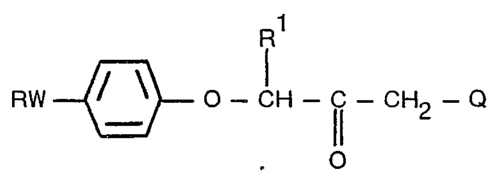 (B')

(C') 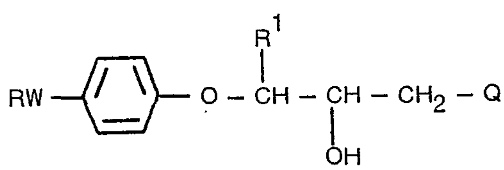

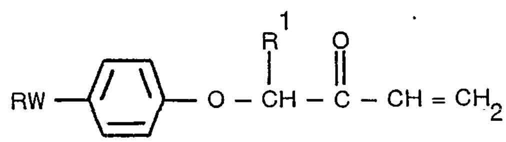 (D')

wherein,

R is

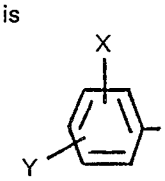 , 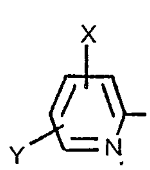 , 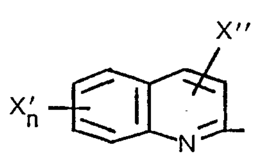 or 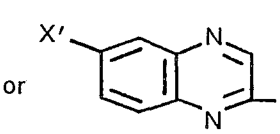 ,

W is O, S, NH or $Ch_2$;

each of X and Y is independently selected from hydrogen, bromo, chloro, fluoro, lower alkyl, lower

alkoxy, lower alkoxycarbonyl, nitro, cyano, trifluoromethyl, chlorodifluoromethyl, fluoromethyl, chloromethyl and difluoromethoxy;

n is 1 or 2;

each of X′ and X″ is independently selected from hydrogen, fluoro, chloro, bromo, iodo, trifluoromethyl, methoxy or nitro, provided that both X′ and X″ cannot be trifluoromethyl, methoxy or nitro;

$R^1$ is hydrogen or lower alkyl;

$R^2$ is lower alkyl;

Q is

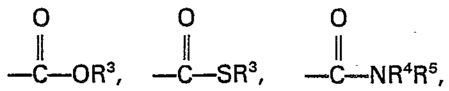

$—CH_2—X^3$ or $—CH_2—OR^6$ in which,

$R^3$ is hydrogen, lower alkyl, lower haloalkyl, lower cyanoalkyl, lower alkenyl, lower haloalkenyl, lower alkynyl, lower alkoxyalkyl, lower dialkylaminoalkyl, lower alkylthioalkyl, lower alkylsulfinylalkyl, lower alkylsulfonylalkyl, tetrahydrofuranylmethyl, tetrahydropyranylmethyl, pyridylmethyl, benzyl, halobenzyl, lower alkylbenzyl, lower alkoxybenzyl, phenyl, halophenyl, lower alkoxyphenyl, lower alkylphenyl, cycloalkyl, cycloalkylalkyl, N-alkylideneamino, sodium, potassium, magnesium or calcium;

each of $R^4$ and $R^5$ is independently selected from hydrogen, lower alkyl, lower hydroxyalkyl, lower alkoxyalkyl or lower alkylsulfonyl;

$X^3$ is bromo, chloro, fluoro or iodo; and

$R^6$ is hydrogen or acyl.

2. A method according to Claim 1 wherein the compound is the methyl or ethyl ester of 4-[4-(4-trifluoromethylphenoxy)phenoxy]-3-oxopentanoic acid.

3. A method according to Claim 1 wherein the compound is of the formula

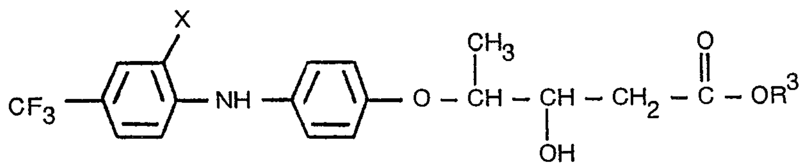

wherein: X is H or nitro, and $R^3$ is H, Na, K or lower alkyl.

4. A method according to Claim 1 wherein the compound is of the formula

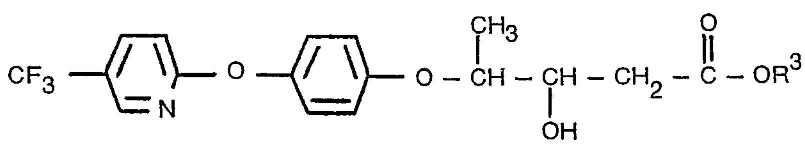

wherein $R^3$ is hydrogen, potassium, sodium or ethyl.

5. A method according to Claim 1 wherein the compound is of the formula

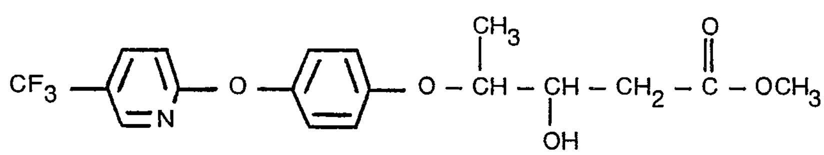

6. A herbicidal composition for the control of weeds comprising a compound of the formula

(A′) 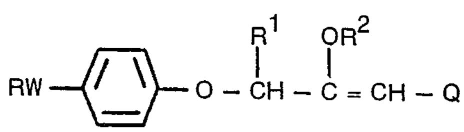

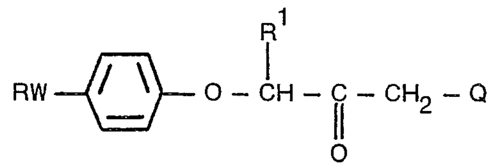 (B′)

(C′) 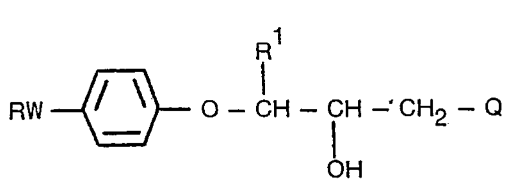

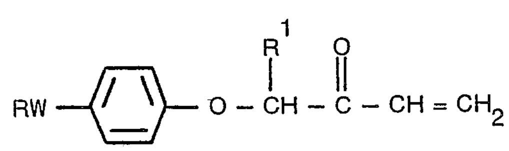 (D′)

wherein,

R is

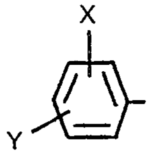 , 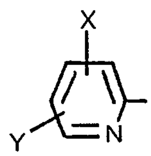 , 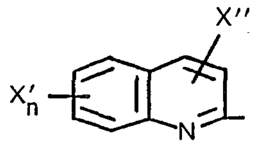 or 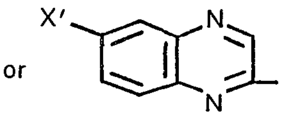 .

W is O, S, NH or $CH_2$;

each of X and Y is independently selected from hydrogen, bromo, chloro, fluoro, lower alkyl, lower alkoxy, lower alkoxycarbonyl, nitro, cyano, trifluoromethyl, chlorodifluoromethyl, fluoromethyl, chloromethyl and difluoromethoxy;

n is 1 or 2;

each of X′ and X″ is independently selected from hydrogen, fluoro, chloro, bromo, iodo, trifluoromethyl, methoxy or nitro, provided that both X′ and X″ cannot be trifluoromethyl, methoxy or nitro;

$R^1$ is hydrogen or lower alkyl;

$R^2$ is lower alkyl;

Q is

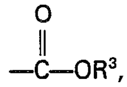, 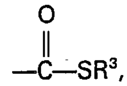, 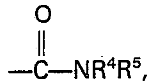,

$—CH_2—X^3$ or $—CH_2—OR^6$ in which,

$R^3$ is hydrogen, lower alkyl, lower haloalkyl, lower cyanoalkyl, lower alkenyl, lower haloalkenyl, lower alkynyl, lower alkoxyalkyl, lower dialkylaminoalkyl, lower alkylthioalkyl, lower alkylsulfinylalkyl, lower alkylsulfonylalkyl, tetrahydrofuranylmethyl, tetrahydropyranylmethyl, pyridylmethyl, benzyl, halobenzyl, lower alkylbenzyl, lower alkoxybenzyl, phenyl, halophenyl, lower alkoxyphenyl, lower alkylphenyl, cycloalkyl, cycloalkylalkyl, N-alkylideneamino, sodium, potassium, magnesium or calcium;

each of $R^4$ and $R^5$ is independently selected from hydrogen, lower alkyl, lower hydroxyalkyl, lower alkoxyalkyl or lower alkylsulfonyl;

$X^3$ is bromo, chloro, fluoro or iodo; and

$R^6$ is hydrogen or acyl.

together with liquid or solid carrier materials.

7. A herbicidal composition according to Claim 6 wherein the compound is the methyl or ethyl ester of 4-[4-(4-trifluoromethylphenoxy)phenoxy]-3-oxopentanoic acid.

8. A herbicidal compound according to Claim 6 wherein the compound is of the formula

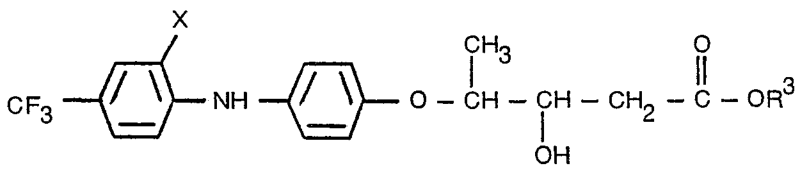

wherein: X is H or nitro, and $R^3$ is H, Na, K or lower alkyl.

9. A herbicidal composition according to Claim 6 wherein the compound is of the formula

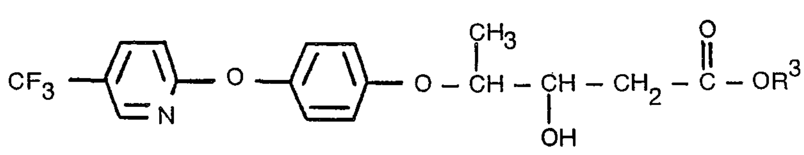

wherein $R^3$ is hydrogen, potassium, sodium or ethyl.

10. A herbicidal composition according to Claim 6 wherein the compound is of the formula

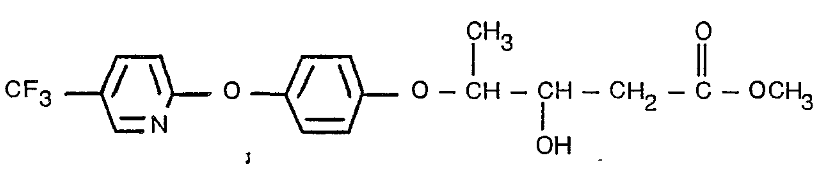

11. A herbicidal composition according to any one of Claims 6 to 10 in the form of dusts, granules, solutions, emulsions, wettable powders or suspension.

**Patentansprüche für die Vertragsstaaten: BE CH DE FR GB IT LI NL**

1. Eine Verbindung ausgewählt aus jenen der folgenden Formeln A', B', C', D'

(A') 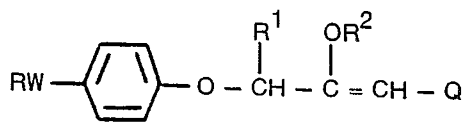 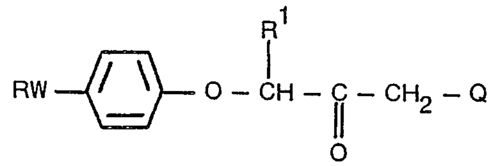 (B')

(C') 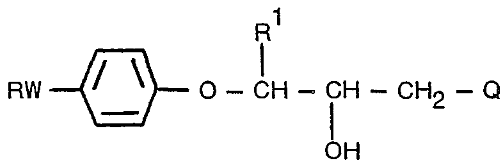 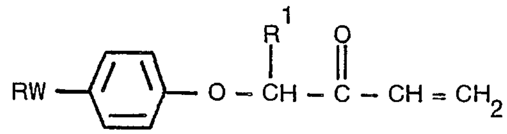 (D')

worin R

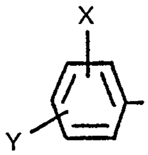 , 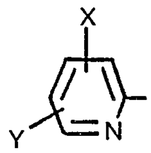 , 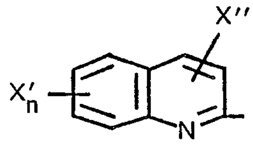 oder 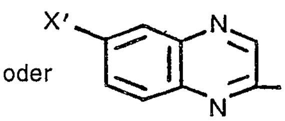 ,

W O, S, NH oder $CH_2$ ist; X und Y jeweils unabhängig voneinander aus Wasserstoff, Brom, Chlor, Fluor, niederem Alkyl, niederem Alkoxy, niederem Alkoxycarbonyl, Nitro, Cyano, Trifluormethyl, Chlordifluormethyl, Fluormethyl, Chlormethyl und Difluormethoxy gewählt werden;

n für 1 oder 2 steht;

X' und X'' jeweils unabhängig voneinander aus Wasserstoff, Fluor, Chlor, Brom, Jod, Trifluormethyl, Methoxy oder Nitro gewählt werden, mit der Maßgabe, daß nicht beide X' und X'' gleichzeitig Trifluormethyl, Methoxy oder Nitro sein können;

$R^1$ Wasserstoff oder ein niederes Alkyl bedeutet;

$R^2$ für ein niederes Alkyl steht;

Q

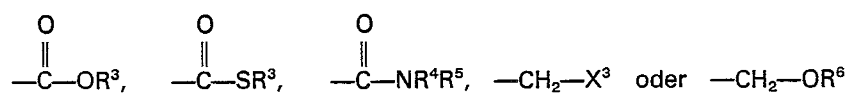

ist,
worin

$R^3$ Wasserstoff, ein niederes Alkyl, ein niederes Halogenalkyl, ein niederes Cyanoalkyl, ein niederes Alkenyl, ein niederes Halogenalkenyl, ein niederes Alkinyl, niederes Alkoxyalkyl, niederes Dialkylaminoalkyl, niederes Alkylthioalkyl, niederes Alkylsulfinylalkyl, niederes Alkylsulfonylalkyl, Tetrahydrofuranylmethyl, Tetrahydropyranylmethyl, Pyridylmethyl, Benzyl, Halogenbenzyl, niederes Alkylbenzyl, niederes Alkoxybenzyl, Phenyl, Halogenphenyl, niederes Alkoxyphenyl, niederes Alkylphenyl, Cycloalkyl, Cycloalkalkyl, N-Alkylidenamino, Natrium, Kalium, Magnesium oder Calcium bedeutet;

$R^4$ und $R^5$ unabhängig jeweils voneinander aus Wasserstoff, niederem Alkyl, niederem Hydroxyalkyl, niederem Alkoxyalkyl oder niederem Alkylsulfonyl gewählt werden;

$X^3$ Brom, Chlor, Fluor oder Jod ist; und

$R^6$ Wasserstoff oder Acyl darstellt.

2. Eine Verbindung der Formel

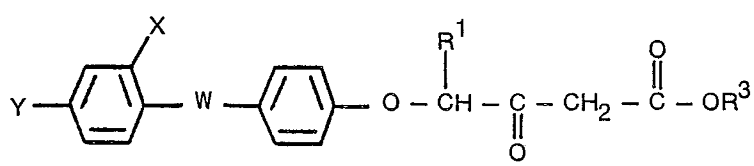

nach Anspruch 1.

3. Eine Verbindung nach Anspruch 2, worin $R^1$ Methyl und W Sauerstoff bedeutet.

4. Eine Verbindung nach Anspruch 3, worin X für Wasserstoff oder Chlor und Y für Trifluormethyl oder Chlor steht.

5. Eine Verbindung nach Anspruch 4, worin $R^3$ ein niederes Alkyl bedeutet.

6. Der Methylester oder Äthylester der 4-[4-(4-Trifluormethylphenoxy)phenoxy]-3-oxopentansäure nach Anspruch 5.

7. Eine Verbindung nach Anspruch 4, worin $R^3$ Wasserstoff, Natrium oder Kalium ist.

8. Eine Verbindung nach Anspruch 4, worin $R^3$ Cyanomethyl, Propargyl, Methoxyäthyl, 3-Methoxy-3-methylbutyl, 2-Chloräthyl, N,N-Dimethylaminomethyl, Äthylthioäthyl, Äthylsulfinyläthyl, Äthylsulfonyläthyl, Benzyl, p-Chlorphenyl, Allyl, 3,3-Dichlorprop-2-enyl, 2-Tetrahydrofuranylmethyl, 2-Tetrahydropyranylmethyl, N-Isopropylidenamino oder N-Cyclohexylidenamino ist.

9. Eine Verbindung nach Anspruch 2, worin W für NH steht, X Nitro bedeutet, Y Trifluormethyl darstellt, $R^1$ Methyl und $R^3$ Wasserstoff, Natrium Kalium oder ein niederes Alkyl ist.

10. Eine Verbindung der Formel

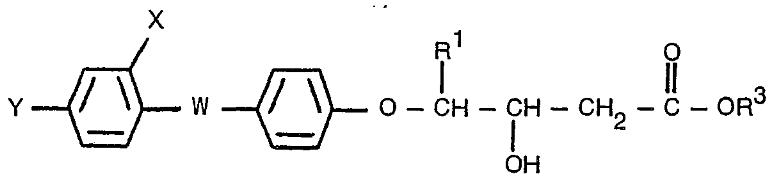

nach Anspruch 1.

11. Eine Verbindung nach Anspruch 10, worin $R^3$ Cyanomethyl, Propargyl, Methoxyäthyl, 3-Methoxy-3-methylbutyl, 2-Chloräthyl, N,N-Dimethylaminomethyl, Äthylthioäthyl, Äthylsulfinyläthyl, Äthylsulfonyläthyl, Benzyl, p-Chlorphenyl, Allyl, 3,3-Dichlorprop-2-enyl, 2-Tetrahydrofuranylmethyl, 2-Tetrahydropyranyl-methyl, N-Isopropylidenamino oder N-Cyclohexylidenamino, $R^1$ Methyl, W Sauerstoff, X Wasserstoff oder Chlor und Y Trifluormethyl oder Chlor bedeutet.

12. Eine Verbindung nach Anspruch 10, worin W für NH steht, X Wasserstoff oder Nitro bedeutet, Y Trifluormethyl darstellt, $R^1$ Methyl und $R^3$ Wasserstoff, Natrium, Kalium oder niederes Alkyl ist.

13. Eine Verbindung nach Anspruch 10, worin W Sauerstoff ist, X für Wasserstoff oder Chlor steht, Y Trifluormethyl darstellt, $R^1$ Methyl und $R^3$ Wasserstoff, Natrium, Kalium oder niederes Alkyl bedeutet.

14. Eine Verbindung nach Anspruch 13, worin $R^3$ Methyl oder Äthyl ist.

15. Eine Verbindung der Formel

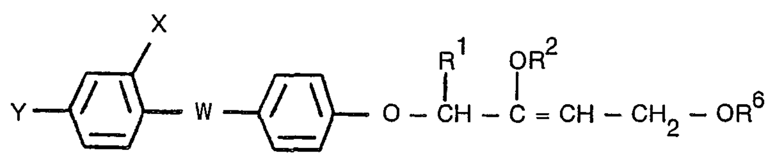

nach Anspruch 1, worin $R^1$ und $R^2$ jeweils Methyl darstellen, W für Sauerstoff steht, X Wasserstoff oder Chlor ist und Y Trifluormethyl oder Chlor bedeutet.

16. Die Verbindung nach Anspruch 15, worin X und $R^6$ jeweils Wasserstoff bedeuten.

17. Eine Verbindung der Formel

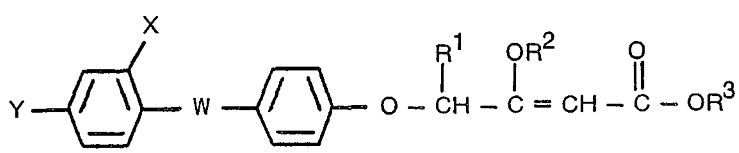

nach Anspruch 1, worin $R^1$ und $R^2$ jeweils Methyl bedeuten, $R^3$ für Wasserstoff, Natrium, Kalium oder niederes Alkyl steht, X Wasserstoff, Chlor oder Nitro darstellt und Y Trifluormethyl oder Chlor ist.

18. Eine Verbindung nach Anspruch 17, worin X für Wasserstoff oder Nitro und W für NH steht.

19. Eine Verbindung nach Anspruch 17, worin X Nitro bedeutet.

20. Eine Verbindung nach Anspruch 17, worin X für Wasserstoff oder Chlor und W für Sauerstoff steht.

21. Eine Verbindung nach Anspruch 18 oder 20, worin $R^3$ Methyl oder Äthyl darstellt.

22. Eine Verbindung der Formel

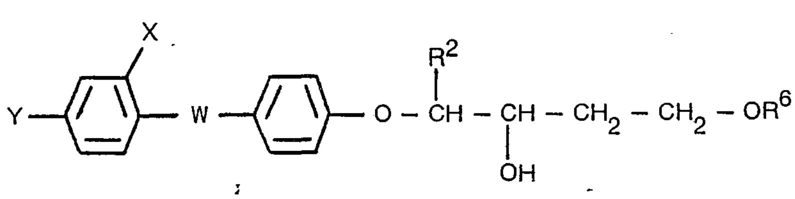

nach Anspruch 1, worin $R^6$ Wasserstoff ist $R^2$ Methyl bedeutet, W für Sauerstoff steht, X Wasserstoff und Y Trifluormethyl oder Chlor darstellt.

23. Eine Verbindung der Formel

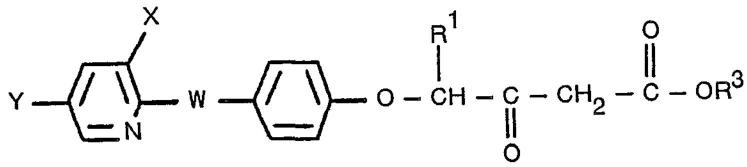

nach Anspruch 1.

24. Eine Verbindung nach Anspruch 23, worin W Sauerstoff, X Wasserstoff, Y Trifluormethyl, $R^1$ Methyl und $R^3$ Wasserstoff, Natrium, Kalium, Methyl oder Äthyl ist.

25. Eine Verbindung nach Anspruch 23, worin W für Sauerstoff, X für Chlor oder Brom, Y für Chlor, $R^1$ für Methyl und $R^3$ für Wasserstoff, Natrium, Kalium, Methyl oder Äthyl steht.

26. Eine Verbindung der Formel

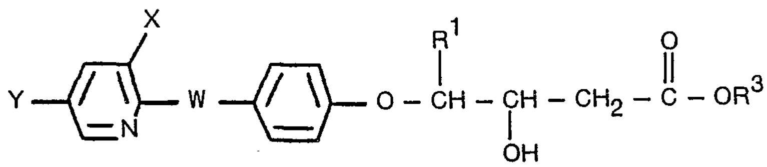

nach Anspruch 1.

27. Eine Verbindung nach Anspruch 26, worin W Sauerstoff, X Wasserstoff, Y Trifluormethyl, $R^1$ Methyl und $R^3$ Wasserstoff, Kalium, Natrium, Methyl oder Äthyl darstellt.

28. Die Verbindung nach Anspruch 26, worin W Sauerstoff, X Wasserstoff, Y Trifluormethyl ist und $R^1$ und $R^3$ beide Methyl bedeuten.

29. Eine Verbindung nach Anspruch 26, worin W Sauerstoff, X Chlor oder Brom, Y Chlor, $R^1$ Methyl und $R^3$ Wasserstoff, Kalium, Natrium, Methyl oder Äthyl ist.

30. Eine herbizide Zusammensetzung, die eine herbizid wirksame Menge einer Verbindung nach Anspruch 1 und ein geeignetes Trägermaterial umfaßt.

31. Eine herbizide Zusammensetzung, die eine herbizid wirksame Menge einer Verbindung nach Anspruch 6 oder Anspruch 12 und ein geeignetes Trägermaterial umfaßt.

32. Ein Verfahren zur Bekämpfung von Unkräutern, das das Aufbringen einer herbizid wirksamen Menge einer Verbindung nach Anspruch 1 auf den Standort der genannten Unkräuter umfaßt.

33. Ein Verfahren zur Bekämpfung von Unkräutern, das das Aufbringen einer herbizid wirksamen Menge einer Verbindung nach Anspruch 6 oder Anspruch 12 auf den Standort der genannten Unkräuter umfaßt.

**Patentansprüche für den Vertragsstaat: AT**

1. Ein Verfahren zur Bekämpfung von Unkräutern, welches das Aufbringen einer Verbindung der Formel

(A′) 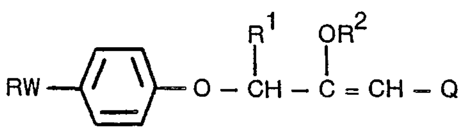

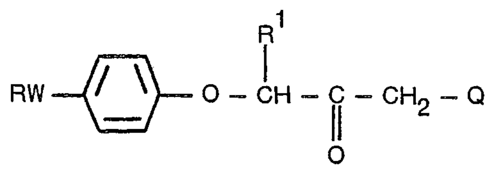 (B′)

(C′) 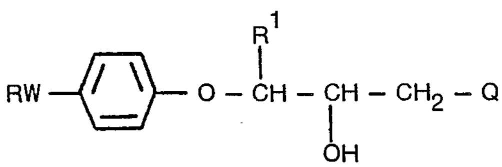

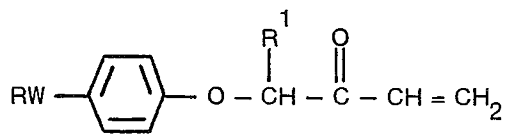 (D′)

auf den Standort der Unkräuter umfaßt, in welcher Formel

R

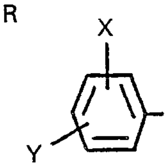 , 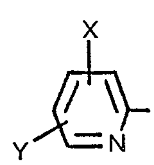 ; 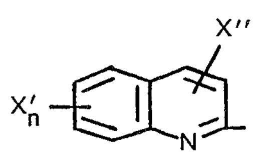 oder 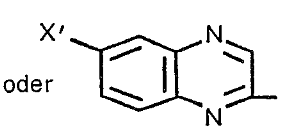 ,

ist,

W für O, S, NH oder $CH_2$ steht;

X und Y jeweils unabhängig voneinander aus Wasserstoff, Brom, Chlor, Fluor, niederem Alkyl, niederem Alkoxy, niederem Alkoxycarbonyl, Nitro, Cyano, Trifluormethyl, Chlordifluormethyl, Fluormethyl, Chlormethyl und Difluormethoxy gewählt werden;

n 1 oder 2 bedeutet;

X′ und X″ jeweils unabhängig voneinander aus Wasserstoff, Fluor, Chlor, Brom, Jod, Trifluormethyl, Methoxy oder Nitro gewählt werden, mit der Maßgabe, daß nicht beide X′ und X″ gleichzeitig Trifluormethyl, Methoxy oder Nitro sein können;

$R^1$ Wasserstoff oder niederes Alkyl darstellt;

$R^2$ niederes Alkyl ist;

Q

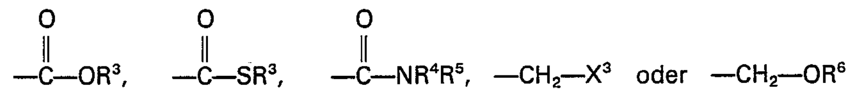

oder

ist,

worin $R^3$ für Wasserstoff, niederes Alkyl, niederes Halogenalkyl, niederes Cyanoalkyl, niederes Alkenyl, niederes Halogenalkenyl, niederes Alkinyl, niederes Alkoxyalkyl, niederes Dialkylaminoalkyl, niederes Alkylthioalkyl, niederes Alkylsulfinylalkyl, niederes Alkylsulfonylalkyl, Tetrahydrofuranylmethyl, Tetrahydropyranylm ethyl, Pyridylmethyl, Benzyl, Halogenbenzyl, niederes Alkylbenzyl, niederes Alkoxybenzyl, Phenyl, Halogenphenyl, niederes Alkoxyphenyl, niederes Alkylphenyl, Cyanoalkyl, Cycloalkylalkyl, N-Alkylidenamino, Natrium, Kalium, Magnesium oder Calcium steht:

$R^4$ und $R^5$ jeweils unabhängig voneinander aus Wasserstoff, niederem Alkyl, niederem Hydroxyalkyl, niederem Alkoxyalkyl oder niederem Alkylsulfonyl gewählt werden;

$X^3$ Brom, Chlor, Fluor oder Jod bedeutet; und

$R^6$ Wasserstoff oder Acyl ist.

2. Ein Verfahren nach Anspruch 1, worin die Verbindung der Methyl- oder Äthylester der 4-[4-(4-Trifluormethylphenoxy)phenoxy]-3-oxopentansäure ist.

3. Ein Verfahren nach Anspruch 1, worin die Verbindung die Formel

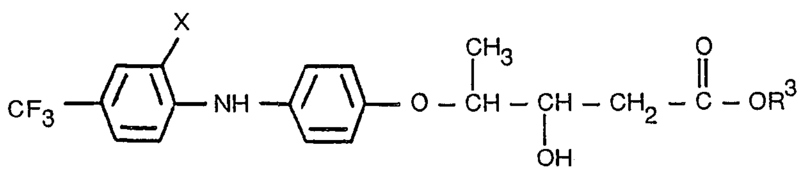

aufweist, worin X für H oder Nitro und $R^3$ für H, Na, K oder Alkyl steht.

4. Ein Verfahren nach Anspruch 1, worin die Verbindung die Formel

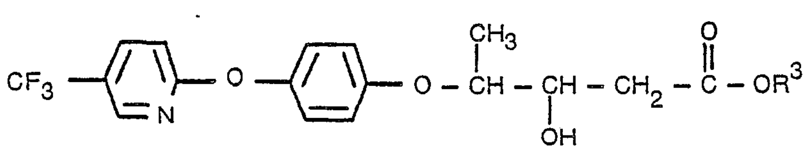

aufweist , worin $R^3$ Wasserstoff, Kalium, Natrium oder Äthyl bedeutet.

5. Ein Verfahren nach Anspruch 1, worin die Verbindung die Formel

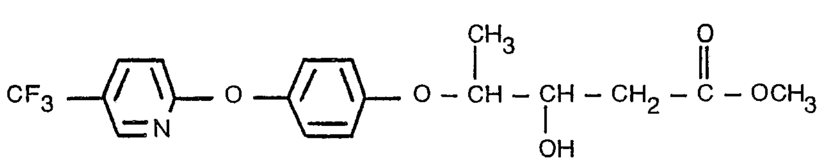

aufweist.

6. Eine herbizide Zusammensetzung für die Bekämpfung von Unkräutern, umfassend eine Verbindung der Formel

(A′) 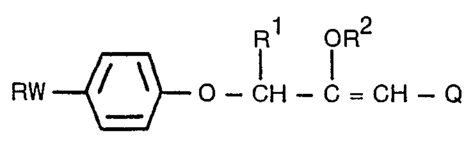

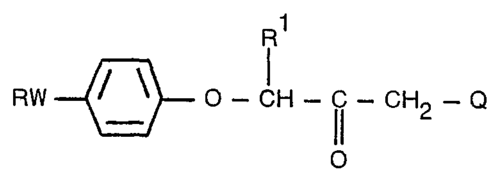 (B′)

(C′) 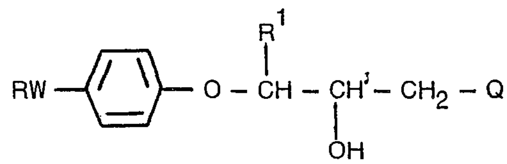

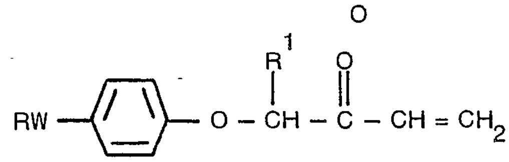 (D′)

worin R

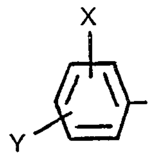, 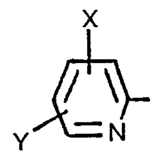, 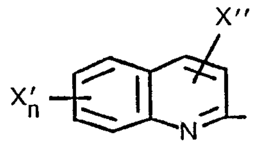 oder 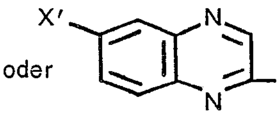,

ist,

W für O, S, NH oder $CH_2$ steht;

X und Y jeweils unabhängig voneinander aus Wasserstoff, Brom, Chlor, Fluor, niederem Alkyl, niederem Alkoxy, niederem Alkoxycarbonyl, Nitro, Cyano, Trifluormethyl, Chlor, Difluormethyl, Fluormethyl, Chlormethyl und Difluormethoxy gewählt werden;

n 1 oder 2 bedeutet;

X' und X'' jeweils unabhängig voneinander aus Wasserstoff, Fluor, Chlor, Brom, Jod, Trifluormethyl, Methoxy oder Nitro gewählt werden, mit der Maßgabe, daß nicht beide X' und X'' gleichzeitig Trifluormethyl, Methoxy oder Nitro sein können;

$R^1$ Wasserstoff oder niederes Alkyl darstellt;

$R^2$ für niederes Alkyl ist;

Q

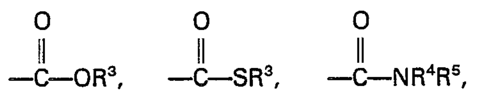, $-CH_2-X^3$ oder $-CH_2-OR^6$

ist, worin

$R^3$ Wasserstoff, niederes Alkyl, niederes Halogenalkyl, niederes Cyanoalkyl, niederes Alkenyl, niederes Halogenalkenyl, niederes Alkinyl, niederes Alkoxyalkyl, niederes Dialkylaminoalkyl, niederes Alkylthioalkyl, niederes Alkylsulfinylalkyl, niederes Alkylsulfonylalkyl, Tetrahydrofuranylmethyl, Tetrahydropyranylmethyl, Pyridylmethyl, Benzyl, Halogenbenzyl, niederes Alkylbenzyl, niederes Alkoxybenzyl, Phenyl, Halogenphenyl, niederes Alkoxyphenyl, niederes Alkylphenyl, Cycloalkyl, Cycloalkylalkyl, N-Alkylidenamino, Natrium, Kalium, Magnesium oder Calcium darstellt;

$R^4$ und $R^5$ jeweils unabhängig voneinander aus Wasserstoff, niederem Alkyl, niederem Hydroxyalkyl, niederem Alkoxyalkyl oder niederem Alkylsulfonyl gewählt werden;

$X^3$ für Brom, Chlor, Fluor oder Jod steht, und

$R^6$ Wasserstoff oder Acyl bedeutet,

zusammen mit flüssigen oder festen Trägermaterialien.

7. Eine herbizide Zusammensetzung nach Anspruch 6, worin die Verbindung der Methyl- oder Äthylester der 4-[4-(4-Trifluormethylphenoxy)phenoxy]-3-oxopentansäure ist.

8. Eine herbizide Zusammensetzung nach Anspruch 6, worin die Verbindung die Formel

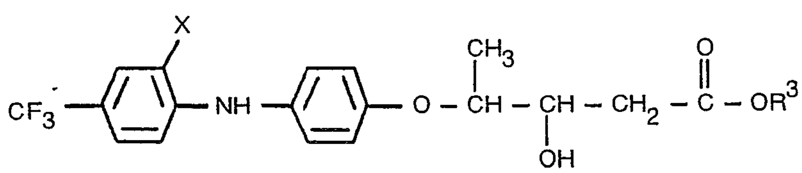

aufweist, worin X für H oder Nitro und $R^3$ für H, Na, K oder niederes Alkyl steht.

9. Eine herbizide Zusammensetzung nach Anspruch 6, worin die Verbindung die Formel

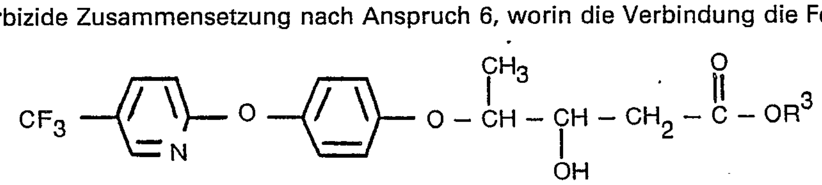

aufweist,

worin $R^3$ Wasserstoff, Kalium, Natrium oder Äthyl bedeutet.

10. Eine herbizide Zusammensetzung nach Anspruch 6, worin die Verbindung die Formel

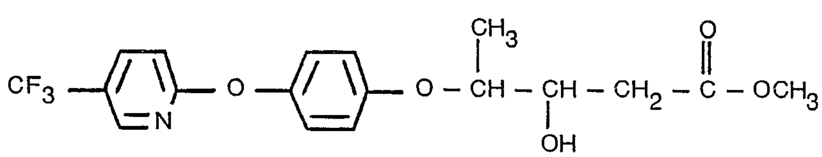

aufweist.

11. Eine herbizide Zusammensetzung nach einem der Ansprüche 6—10 in Form von Stäuben, Granulaten, Lösungen, Emulsionen, benetzbaren Pulvern oder Suspensionen.

**Revendications pour les Etats contractants: BE CH DE FR GB IT LI NL**

1. Composé choisi parmi ceux des formules A', B', C' et D' qui suivent:

(A') 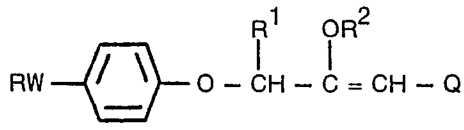 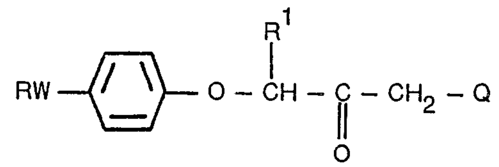 (B')

(C') 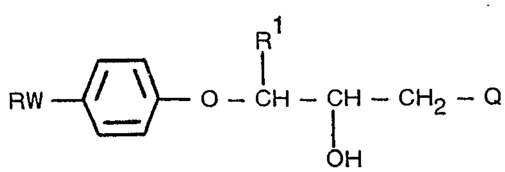 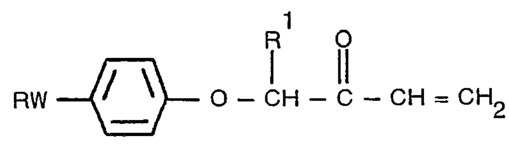 (D')

où,

R est

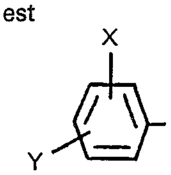 , 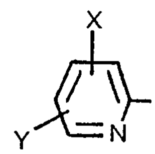 , 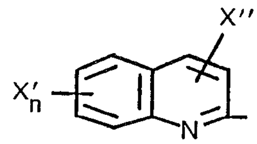 ou 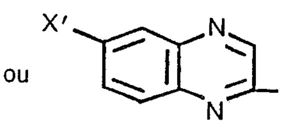 ,

W est O, S, NH ou $CH_2$;

chacun de X et Y est indépendamment choisi parmi l'hydrogène, bromo, chloro, fluoro, alcoyle inférieur, alcoxy inférieur, alcoxycarbonyle inférieur, nitro, cyano, trifluorométhyle, chlorodifluorométhyle, fluorométhyle, chlorométhyle et difluorométhoxy;

n est 1 ou 2;

chacun de X' et X'' est indépendamment choisi parmi l'hydrogène, fluoro, chloro, bromo, iodo, trifluorométhyle, méthoxy ou nitro à condition que X' et X'' ne puissent être tous deux trifluorométhyle, méthoxy ou nitro;

$R^1$ est hydrogène ou alcoyle infèrieur;

$R^2$ est alcoyle inférieur;

Q est

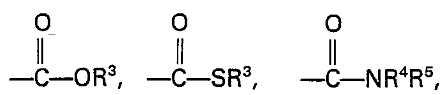

$—CH_2—X^3$ ou $—CH_2—OR^6$ où,

$R^3$ est hydrogène, alcoyle inférieur, haloalcoyle inférieur, cyanoalcoyle inférieur, alkényle inférieur, haloalkényle inférieur, alkynyle inférieur, alcoxyalcoyle inférieur, dialcoyle inférieur-aminoalcoyle, alcoyle inférieur-thioalcoyle, alcoyle inférieur-sulfinylalcoyle, alcoyle inférieur-sulfonylalcoyle, tétrahydrofuranylméthyle, tétrahydropyranylméthyle, pyridylméthyle, benzyle, halobenzyle, alcoyle inférieur-benzyle, alcoxy inférieur-benzyle, phényle, halophényle, alcoxy inférieur-phényle, alcoyle inférieur-phényle, cycloalcoyle, cycloalkalcoyle, N-alcoylidèneamino, sodium, potassium, magnésium ou calcium;

chacun de $R^4$ et $R^5$ est indépendamment choisi parmi l'hydrogène, alcoyle inférieur, hydroxyalcoyle inférieur, alcoxyalcoyle inférieur ou alcoyle inférieur-sulfonyle;

$X^3$ est bromo, chloro, fluoro ou iodo; et

$R^6$ est hydrogène ou acyle.

2. Composé de formule

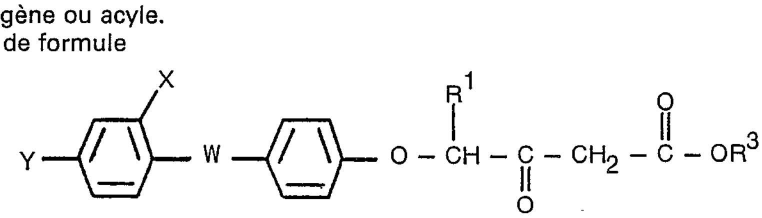

selon la revendication 1.

3. Composé selon la revendication 2 où $R^1$ est du méthyle et W est de l'oxygène.

4. Composé selon la revendication 3 où X est hydrogène ou chloro et Y est trifluorométhyle ou chloro.

5. Composé selon la revendication 4 où $R^3$ est un alcoyle inférieur.

6. Ester méthylique ou ester éthylique de l'acide 4-[4-(4-trifluorométhylphénoxy)phénoxy]-3-oxo-pentanoique selon la revendication 5.

7. Composé selon la revendication 4 où $R^3$ est de l'hydrogène, du sodium ou du potassium.

8. Composé selon la revendication 4 où $R^3$ est cyanométhyle, propargyle, méthoxyéthyle, 3-méthoxy-3-méthylbutyle, 2-chloroéthyle, N,N-diméthylaminoéthyle, éthylthioéthyle, éthylsulfinyléthyle, éthylsulfonyléthyle, benzyle, p-chlorophényle, allyle, 3,3-dichloroprop-2-ényle, 2-tétrahydrofuranylméthyle, 2-tétrahydropyranylméthyle, N-isopropylidèneamino ou N-cyclohexylidèneamino.

9. Composé selon la revendication 2 où W est NH, X est nitro, Y est trifluorométhyle, $R^1$ est méthyle et $R^3$ est de l'hydrogène, du sodium, du potassium ou un alcoyle inférieur.

10. Composé de formule

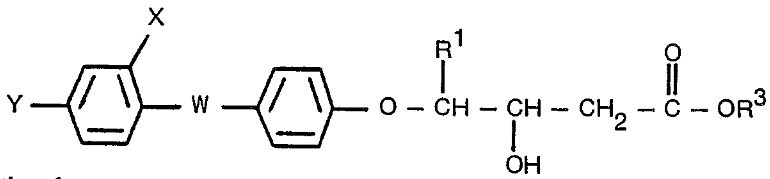

selon la revendication 1.

11. Composé selon la revendication 10, où $R^3$ est cyanométhyle, propargyle, méthoxyéthyle, 3-méthoxy-3-méthylbutyle, 2-chloroéthyle, N,N-diméthylaminométhyle, éthylthioéthyle, éthylsulfinyléthyle, éthylsulfonyléthyle, benzyle, p-chlorophényle, allyle, 3,3-dichloroprop-2-ényle, 2-tétrahydrofuranylméthyle, 2-tétrahydropyranyl-méthyle, N-isopropylidèneamino ou N-cyclohexylidèneamino, $R^1$ est méthyle, W est oxygène, X est hydrogène ou chloro et Y est trifluorométhyle ou chloro.

12. Composé selon la revendication 10 où W est NH, X est hydrogène ou nitro, Y est trifluorométhyle, $R^1$ est méthyle et $R^3$ est hydrogène, sodium, potassium ou alcoyle inférieur.

13. Composé selon la revendication 10 où W est de l'oxygène, X est hydrogène ou chloro, Y est trifluorométhyle, $R^1$ est méthyle et $R^3$ est hydrogène, sodium, potassium ou alcoyle inférieur.

14. Composé selon la revendication 13 où $R^3$ est méthyle ou éthyle.

15. Composé de formule

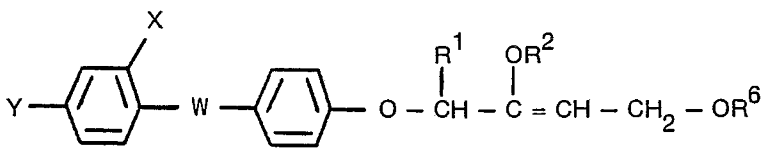

selon la revendication 1, où chacun de $R^1$ et $R^2$ est méthyle, W est de l'oxygène, X est de l'hydrogène ou chloro et Y est trifluorométhyle ou chloro.

16. Composé selon la revendication 15 où chacun de X et $R^6$ est de l'hydrogène.

17. Composé de formule

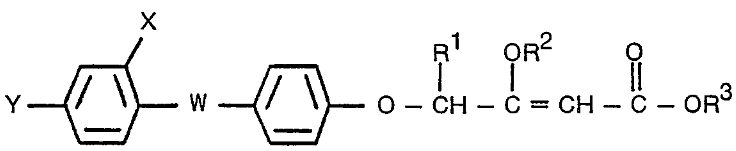

selon la revendication 1, où chacun de $R^1$ et $R^2$ est méthyle, $R^3$ est hydrogène, sodium, potassium ou alcoyle inférieur, X est hydrogène, chloro ou nitro et Y est trifluorométhyle ou chloro.

18. Composé selon la revendication 17 où X est hydrogène ou nitro et W est NH.

19. Composé selon la revendication 17 où X est nitro.

20. Composé selon la revendication 17 où X est hydrogène ou chloro et W est oxygène.

21. Composé selon la revendication 18 ou la revendication 20 où $R^3$ est méthyle ou éthyle.

22. Composé de formule

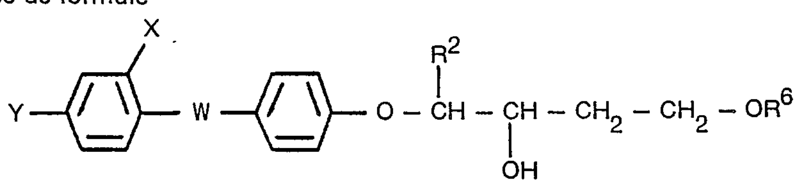

selon la revendication 1 où $R^6$ est hydrogène, $R^2$ est méthyle, W est oxygène, X est hydrogène et Y est trifluorométhyle ou chloro.

23. Composé de formule

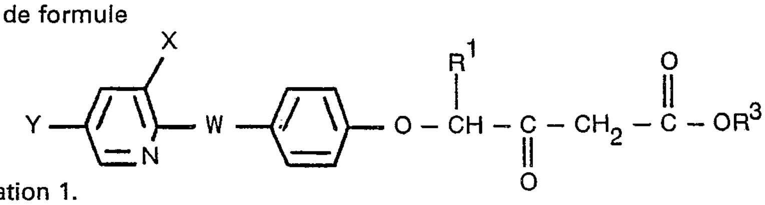

selon la revendication 1.

24. Composé selon la revendication 23 où W est de l'oxygène, X est de l'hydrogène, Y est trifluorométhyle, $R^1$ est méthyle et $R^3$ est de l'hydrogène, du sodium, du potassium, du méthyle ou de l'éthyle.

25. Composé selon la revendication 23, où W est de l'oxygène, X est chloro ou bromo, Y est chloro, $R^1$ est méthyle et $R^3$ est hydrogène, sodium, potassium, méthyle ou éthyle.

26. Composé de formule

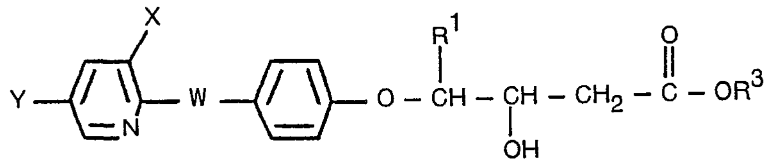

selon la revendication 1.

27. Composé selon la revendication 26 où W est de l'oxygène, X est de l'hydrogène, Y est trifluorométhyle, $R^1$ 'est du méthyle et $R^3$ est de l'hydrogène, du potassium, du sodium, du méthyle ou de l'éthyle.

28. Composé selon la revendication 26 où W est de l'oxygène, X est de l'hydrogène Y est du trifluorométhyle et $R^1$ et $R^3$ sont tous deux du méthyle.

29. Composé selon la revendication 26 où W est de l'oxygène, X est chloro ou bromo, Y est chloro, $R^1$ est du méthyle et $R^3$ est de l'hydrogène, du potassium, du sodium, du méthyle ou de l'éthyle.

30. Composition herbicide qui contient une quantité herbicidement efficace d'un composé selon la revendication 1 et un matériau de support approprié.

31. Composition herbicide qui comprend une quantité herbicidement efficace d'un composé selon la revendication 6 ou la revendication 12 et un matériau de support approprié.

32. Méthode pour le contrôle des mauvaises herbes qui consiste à appliquer, au lieu desdites mauvaises herbes, une quantité herbicidement efficace d'un composé selon la revendication 1.

33. Méthode pour le contrôle des mauvaises herbes qui consiste à appliquer au lieu desdites mauvaises herbes, une quantité herbicidement efficace d'un composé selon la revendication 6 ou la revendication 12.

**Revendications pour l'Etat contractant: AT**

1. Méthode pour le contrôle des mauvaises herbes qui consiste à appliquer, au lieu des mauvaises herbes, un composé de formule:

(A') 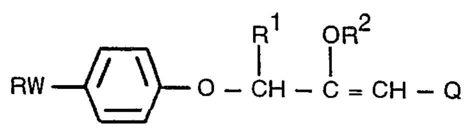

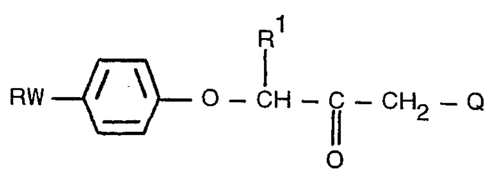 (B')

(C') 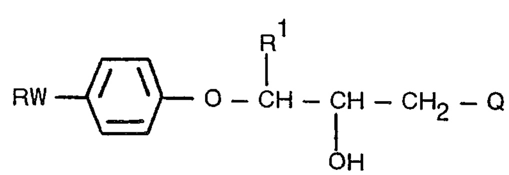

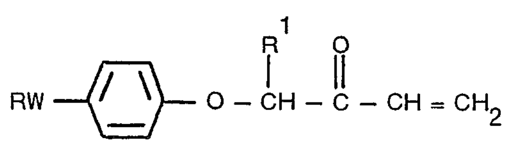 (D')

où,

R est

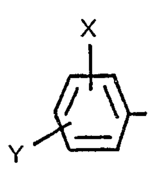 , 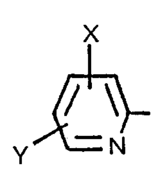 , 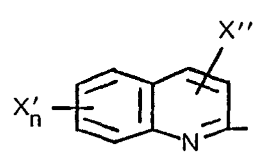 ou 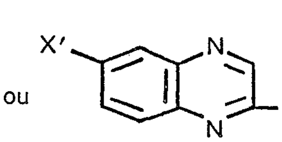 ,

W est O, S, NH ou $CH_2$;

chacun de X et Y est indépendamment choisi parmi l'hydrogène, bromo, chloro, fluoro, alcoyle inférieur, alcoxy inférieur, alcoxycarbonyle inférieur, nitro, cyano, trifluorométhyle, chlorodifluorométhyle, fluorométhyle, chlorométhyle et difluorométhoxy;

n est 1 ou 2;

chacun de X' et X'' est indépendamment choisi parmi l'hydrogène, fluoro, chloro, bromo, iodo, trifluorométhyle, méthoxy ou nitro à condition que X' et X'' ne puissent être tous deux trifluorométhyle, méthoxy ou nitro;

$R^1$ est hydrogène ou alcoyle infèrieur;
$R^2$ est alcoyle inférieur;
Q est

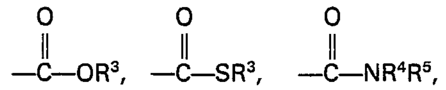

$—CH_2—X^3$ ou $—CH_2—OR^6$ où,

$R^3$ est hydrogène, alcoyle inférieur, haloalcoyle inférieur, cyanoalcoyle inférieur, alkényle inférieur, haloalkényle inférieur, alkynyle inférieur, alcoxyalcoyle inférieur, dialcoyle inférieur-aminoalcoyle, alcoyle inférieur-thioalcoyle, alcoyle inférieur-sulfinylalcoyle, alcoyle inférieur-sulfonylalcoyle, tétrahydrofuranylméthyle, tétrahydropyranylméthyle, pyridylméthyle, benzyle, halobenzyle, alcoyle inférieur-benzyle, alcoxy inférieur-benzyle, phényle, halophényle, alcoxy inférieur-phényle, alcoyle inférieur-phényle, cycloalcoyle, cycloalkylalcoyle, N-alcoylidèneamino, sodium, potassium, magnésium ou calcium;

chacun de $R^4$ et $R^5$ est indépendamment choisi parmi l'hydrogène, alcoyle inférieur, hydroxyalcoyle inférieur, alcoxyalcoyle inférieur ou alcoyle inférieur-sulfonyle;

$X^3$ est bromo, chloro, fluoro ou iodo; et

$R^6$ est hydrogène ou acyle.

2. Méthode selon la revendication 1 où le composé est l'ester méthylique ou éthylique de l'acide 4-[4-(4-trifluorométhylphénoxy)phénoxy]-3-oxopentanoique.

3. Méthode selon la revendication 1 où le composé est de formule

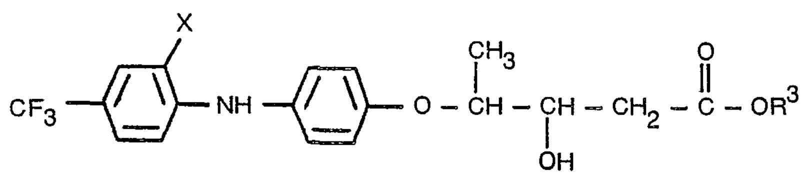

où:

X est H ou nitro, et

$R^3$ est H, Na, K ou alcoyle inférieur.

4. Méthode selon la revendication 1 où le composé a pour formule

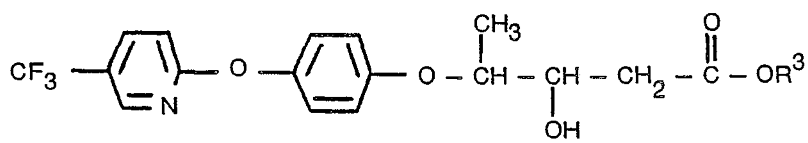

où $R^3$ est de l'hydrogène, du potassium, du sodium ou de l'éthyle.

5. Méthode selon la revendication 1 où le composé a pour formule

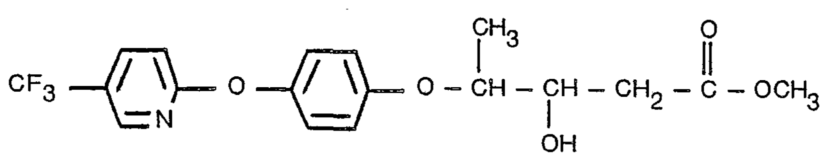

6. Composition herbicide pour le contrôle des mauvaises herbes comprenant un composé de formule

(A') 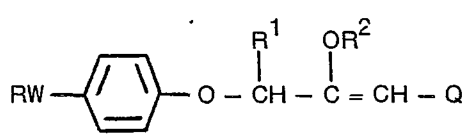

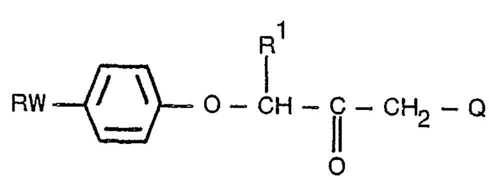 (B')

(C') 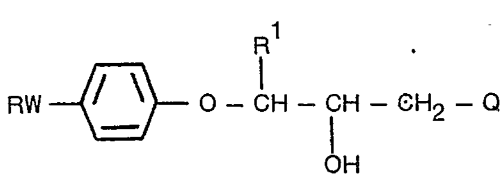

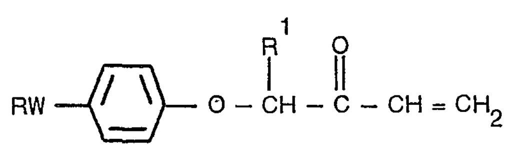 (D')

où,

R est

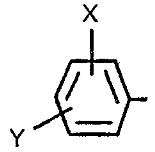 , 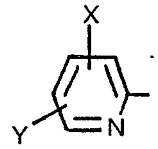 , 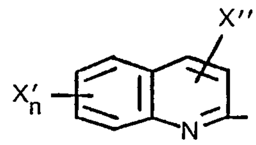 ou 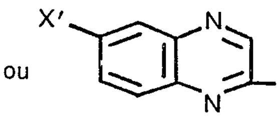 ,

W est O, S, NH ou $CH_2$;

chacun de X et Y est indépendamment choisi parmi l'hydrogène, bromo, chloro, fluoro, alcoyle inférieur, alcoxy inférieur, alcoxycarbonyle inférieur, nitro, cyano, trifluorométhyle, chlorodifluorométhyle, fluorométhyle, chlorométhyle et difluorométhoxy;

n est 1 ou 2;

chacun de X' et X'' est indépendamment choisi parmi l'hydrogène, fluoro, chloro, bromo, iodo, trifluorométhyle, méthoxy ou nitro à condition que X' et X'' ne puissent être tous deux trifluorométhyle, méthoxy ou nitro;

$R^1$ est hydrogène ou alcoyle infèrieur;

$R^2$ est alcoyle inférieur;

Q est

$$-\overset{\overset{\displaystyle O}{\|}}{C}-OR^3,\quad -\overset{\overset{\displaystyle O}{\|}}{C}-SR^3,\quad -\overset{\overset{\displaystyle O}{\|}}{C}-NR^4R^5,$$

$-CH_2-X^3$ ou $-CH_2-OR^6$ où,

$R^3$ est hydrogène, alcoyle inférieur, haloalcoyle inférieur, cyanoalcoyle inférieur, alkényle inférieur, haloalkényle inférieur, alkynyle inférieur, alcoxyalcoyle inférieur, dialcoyle inférieur-aminoalcoyle, alcoyle inférieur-thioalcoyle, alcoyle inférieur-sulfinylalcoyle, alcoyle inférieur-sulfonylalcoyle, tétrahydrofuranylméthyle, tétrahydropyranylméthyle, pyridylméthyle, benzyle, halobenzyle, alcoyle inférieur-benzyle, alcoxy inférieur-benzyle, phényle, halophényle, alcoxy inférieur-phényle, alcoyle inférieur-phényle, cycloalcoyle, cycloalkylalcoyle, N-alcoylidèneamino, sodium, potassium, magnésium ou calcium;

chacun de $R^4$ et $R^5$ est indépendamment choisi parmi l'hydrogène, alcoyle inférieur, hydroxyalcoyle inférieur, alcoxyalcoyle inférieur ou alcoyle inférieur-sulfonyle;

$X^3$ est bromo, chloro, fluoro ou iodo; et

$R^6$ est hydrogène ou acyle,

avec des matériaux de support liquides ou solides.

7. Composition herbicide selon la revendication 6 où le composé est l'ester méthylique ou éthylique de l'acide 4-[4-(4-trifluorométhylphénoxy)phénoxy]-3-oxopentanoique.

8. Composé herbicide selon la revendication 6 où le composé a pour formule

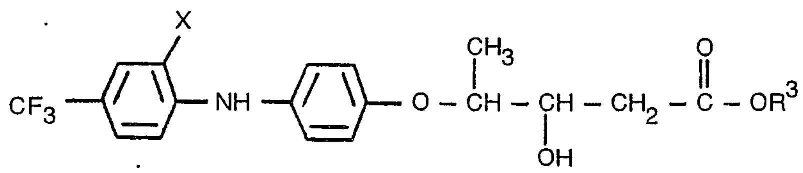

où:

X est H ou nitro, et

$R^3$ est H, Na, K ou alcoyle inférieur.

9. Composition herbicide selon la revendication 6 où le composé a pour formule

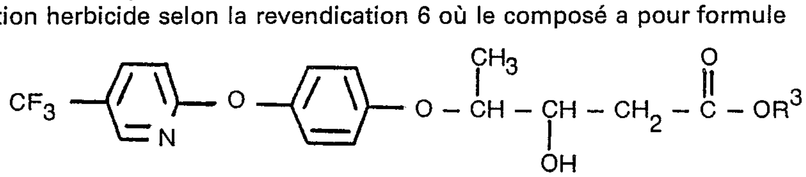

où $R^3$ est de l'hydrogène, du potassium, du sodium ou de l'éthyle.

10. Composition herbicide selon la revendication 6 où le composé a pour formule

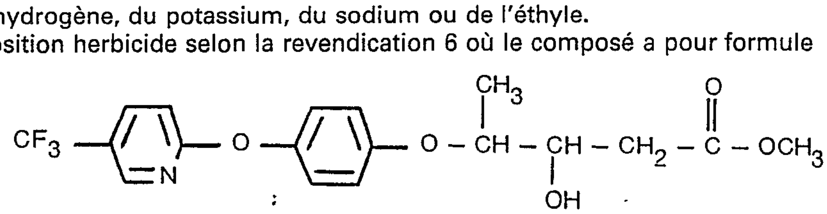

11. Composition herbicide selon l'une quelconque des revendications 6 à 10 sous la forme de poussières, granules, solutions, émulsions, poudres mouillables ou suspension.